# EUROPEAN PATENT APPLICATION

(11) **EP 2 158 834 A1**
(43) Date of publication of application: **03.03.2010**
(21) Application number: 07767225.1
(22) Date of filing: 20.06.2007
(51) Int. Cl.: A61B 1/00, A61B 5/07

(54) **ENDOSCOPE SYSTEM, IMAGING SYSTEM, AND IMAGE PROCESSING DEVICE**

(71) Applicant: Olympus Medical Systems Corp., Tokyo 151-0072 (JP)
(72) Inventor: TANAKA, Hideki, Tokyo 151-0072 (JP); HASEGAWA, Jun, Tokyo 151-0072 (JP); NAKAMURA, Toshio, Tokyo 151-0072 (JP); USHIYAMA, Akio, Tokyo 151-0072 (JP); CHIBA, Atsushi, Tokyo 151-0072 (JP)
(74) Representative: von Hellfeld, Axel
(86) International application number: PCT/JP2007/062386
(87) International publication number: WO 2008/155828

(57) **Abstract**

An endoscope system includes: an endoscope for picking up an image in a body cavity by an image pickup apparatus provided in a distal end of an insertion portion; a position detecting apparatus for detecting, based on luminal information acquired by the image pickup apparatus, position information used for inserting the distal end of the insertion portion; a recording apparatus for recording, in a time-sequential manner, the position information detected by the position detecting apparatus; a determining apparatus for determining whether or not the detecting operation of the position information performed by the position detecting apparatus satisfies a set condition; and a direction calculating apparatus for, when the determination result shows that the set condition is not satisfied, reading out the position information recorded in the recording apparatus and outputting information on a direction in which the distal end of the insertion portion is to be inserted.

## Description

### Technical Field

The present invention relates to an endoscope system, an image pickup system and an image processing apparatus for acquiring an image inside a body cavity to examine and diagnose inside of the body cavity.

### Background Art

In recent years, endoscopes have been widely used to examine and diagnose inside of a body cavity. When endoscopes are used, it is desirable that an insertion portion is smoothly inserted into a body cavity.

For example, Japanese Patent Application Laid-Open Publication No. 2003-93328 as a first prior art example discloses to detect a direction in which a distal end portion of an insertion portion is to be inserted, that is, a target position, based on an endoscopic image and set the direction of the target position as the insertion direction.

In addition, Japanese Patent Application Laid-Open Publication No. 2006-116298 as a second prior art example discloses a bending controlling apparatus for controlling bending at the time of insertion by selecting a first bending controlling method based on an image picked by an endoscope and a second bending controlling method based on a detected image of an endoscope insertion shape and a CT image.

However, in the first prior art example, when a dark part corresponding to a running direction of a body cavity or a lumen cannot be detected as an endoscopic image or the dark part disappears and the endoscopic image shows the state where the mucosal surface is picked up, it is difficult to select the insertion direction. In this case, in the fourth embodiment of the first prior art, when the dark part as a target position disappears to outside of the image, the insertion direction is shown based on the disappearing direction of the dark part.

However, the information before the dark part disappears is not stored, so that it is difficult to accurately show the insertion direction.

Furthermore, if the endoscope is twisted during the period after the dark part disappeared until the insertion direction is shown, also the direction of the target position is rotated and moved, so that there is a disadvantage that the direction in which the dark part has disappeared cannot reproduced.

In addition, in the second prior art example, when the bending control method based on an endoscopic image is selected, if the dark part disappears, it is difficult to show the bending direction as the insertion direction similarly as in the case of the first prior art example.

The present invention has been achieved in view of the above-described points, and an object of the present invention is to provide an endoscope system, an image pickup system and an image processing apparatus for detecting an insertion direction and a bending direction so as to be able to smoothly acquire an image for examination and diagnosis inside of a body cavity using an endoscope and the like.

### Disclosure of Invention

### Means for Solving the Problem

An endoscope system according to the present invention comprises: an endoscope for picking up an image in a body cavity by image pickup means provided in a distal end of an insertion portion; position detecting means for detecting, based on luminal information acquired by the image pickup means, position information used for inserting the distal end of the insertion portion; recording means for recording, in a time-sequential manner, the position information detected by the position detecting means; determining means for determining whether or not the detecting operation of the position information performed by the position detecting means satisfies a set condition; and direction calculating means for, when the determination result shows that the set condition is not satisfied, reading out the position information recorded in the recording means and outputting information on a direction in which the distal end of the insertion portion is to be inserted.

An image pickup system according to the present invention comprises: an image pickup section provided in an insertion body configured to be inserted in a body cavity, for picking up an image in the body cavity; luminal information detecting means for detecting luminal information corresponding to a running direction of the body cavity based on the image picked up by the image pickup section; recording means for recording, in a time-sequential manner, luminal information detected by the luminal information detecting means; estimating means for estimating a position and a direction of the image pickup section; determining means for determining whether or not the detecting operation of the luminal information performed by the luminal information detecting means satisfies a set condition; direction calculating means for, when the determining means determines that the condition is not satisfied, reading out the luminal information recorded in the recording means and calculating information on a direction in which the insertion body is moved based on the luminal information and an estimation result acquired by the estimating means; and controlling means for controlling the direction in which the insertion body is moved, based on the information calculated by the direction calculating means.

An image processing apparatus according to the present invention comprises: an inputting section for inputting an endoscopic image picked up by image pickup means provided in a distal end portion of an insertion portion configured to be inserted in a body cavity; position detecting means for performing a processing of detecting, from the endoscopic image, position information used for introducing the distal end of the insertion portion; recording means for recording, in a time-sequential manner, the position information detected by the position detecting means; determining means for performing determining processing as to whether or not the processing of detecting the position information performed by the position detecting means satisfies a set condition; and calculating means for, when the determining means determines that the condition is not satisfied, reading out position information recorded in the recording means and outputting information on a direction in which the distal end of the insertion portion is inserted.

### Brief Description of the Drawings

FIG. 1 is a view showing an overall configuration of an endoscope system according to a first embodiment of the present invention.
FIG. 2 is an overall configurational view showing a specific configuration in FIG. 1.
FIG. 3 is a view showing a configuration of an amount-of-twist detecting unit.
FIG. 4 is a block diagram showing a configuration of a functional block of a PC main body.
FIG. 5 is a block diagram showing a functional configuration of bending control by a main processing section.
FIG. 6A is a view showing a state where an insertion portion of an endoscope is inserted in a large intestine.
FIG. 6B is a view showing an exemplary image which can be acquired in a state where a dark part exists in the image in the case shown in FIG. 6A.
FIG. 7A is a view showing a state where the insertion portion of the endoscope is inserted in the large intestine.
FIG. 7B is a view showing an exemplary image from which the dark part has disappeared in the case shown in FIG. 7A.
FIG. 8A is a view showing a display example in which a bending direction and the like are displayed.
FIG. 8B is an endoscopic image.
FIG. 9 is a view showing an operation of bending control for bending a bending portion in a direction of the dark part.
FIG. 10 is a flowchart showing an operation content of the main processing section of the present embodiment.
FIG. 11 is an operation illustration diagram showing information on absolute amounts of twist and corresponding intra-image target positions which are stored in a ring buffer in order of time.
FIG. 12 is an operation illustration diagram showing information on the absolute amounts of twist and corresponding shapes of the endoscope which are stored in the ring buffer in order of time.
FIG. 13 is a view showing an overall configuration of an endoscope system according to a first modified example of the first embodiment.
FIG. 14 is a view showing an overall configuration of an endoscope system according to a second modified example of the first embodiment.
FIG. 15 is a block diagram showing a functional configuration of a main processing section in the second modified example.
FIG. 16 is a flowchart showing an operation content of the main processing section of the second modified example.
FIG. 17 is a view showing an overall configuration of an endoscope system according to a third modified example of the first embodiment.
FIG. 18 is a flowchart showing an operation content of a main processing section of a third modified example.
FIG. 19 is a view showing an overall configuration of an endoscope system according to a fourth modified example of the first embodiment.
FIG. 20 is a view showing a configuration of a main part according a second embodiment of the present invention.
FIG. 21 is an overall configurational view of a capsule medical system according to the second embodiment.
FIG. 22 is a more detailed block diagram of the capsule medical system in FIG. 21.
FIG. 23 is an illustration diagram showing a side surface of a capsule main body.
FIG. 24 is a concept view showing an applied rotational magnetic field and how the capsule main body is operated by the rotational magnetic field.
FIG. 25 is a concept view showing a vibration magnetic field (couple generating magnetic field) applied to the rotational magnetic field in FIG. 24 and how the capsule main body is operated by the vibration magnetic field (couple generating magnetic field).
FIG. 26 is a view showing specific position information and the like recorded in recording means in a time-sequential manner.
FIG. 27 is a view showing exemplary images acquired by the image pickup means in the capsule main body.
FIG. 28 is a view showing the states of the capsule main body and the lumen corresponding to the images in FIG. 27.
FIG. 29 is a flowchart showing an operation content of the second embodiment.
FIG. 30 is a view showing a configuration of a main part of a modified example of the second embodiment.
FIG. 31 is a flowchart showing a part of operation content of the modified example.

### Best Mode for Carrying Out the Invention

Hereinafter, embodiments of the present invention will be described with reference to the drawings.

### First Embodiment

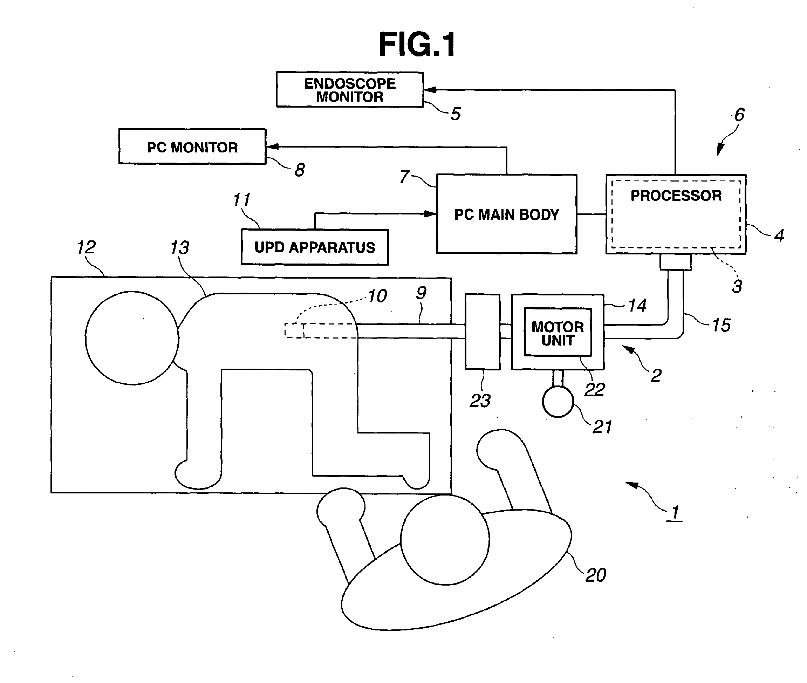

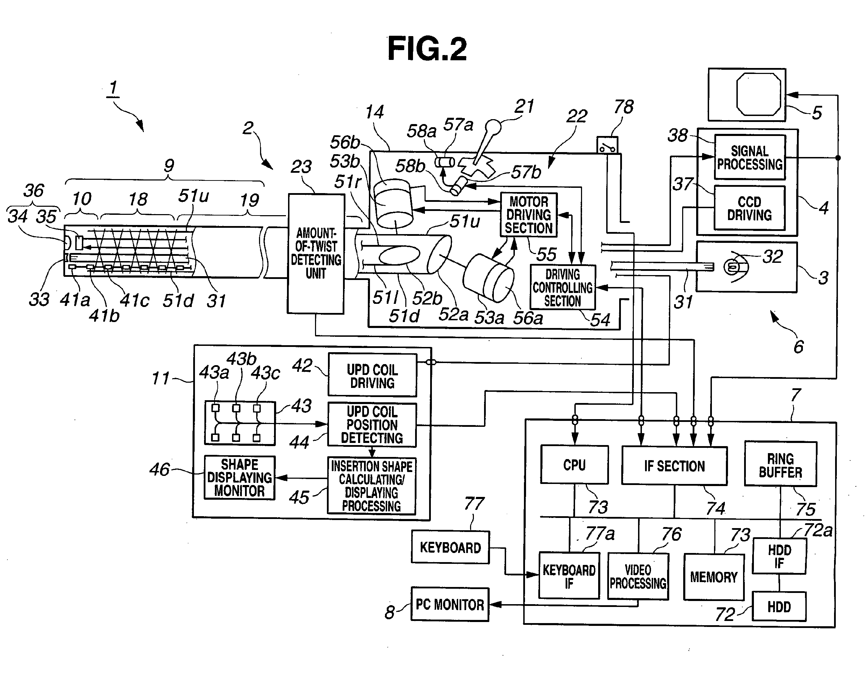

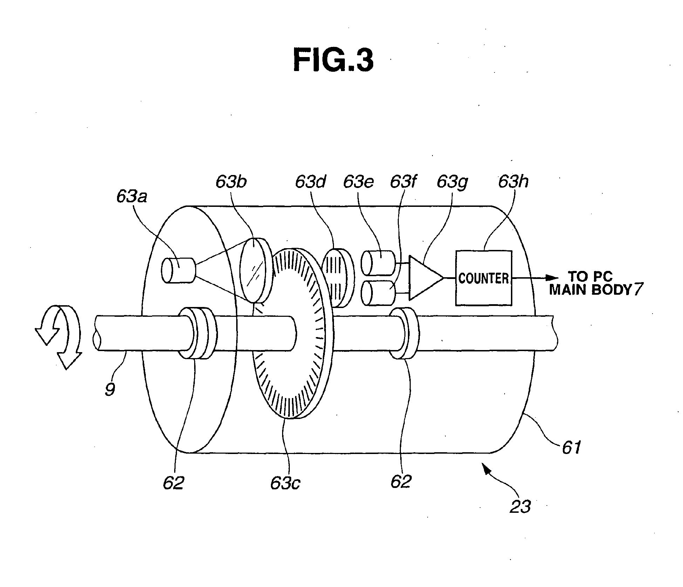

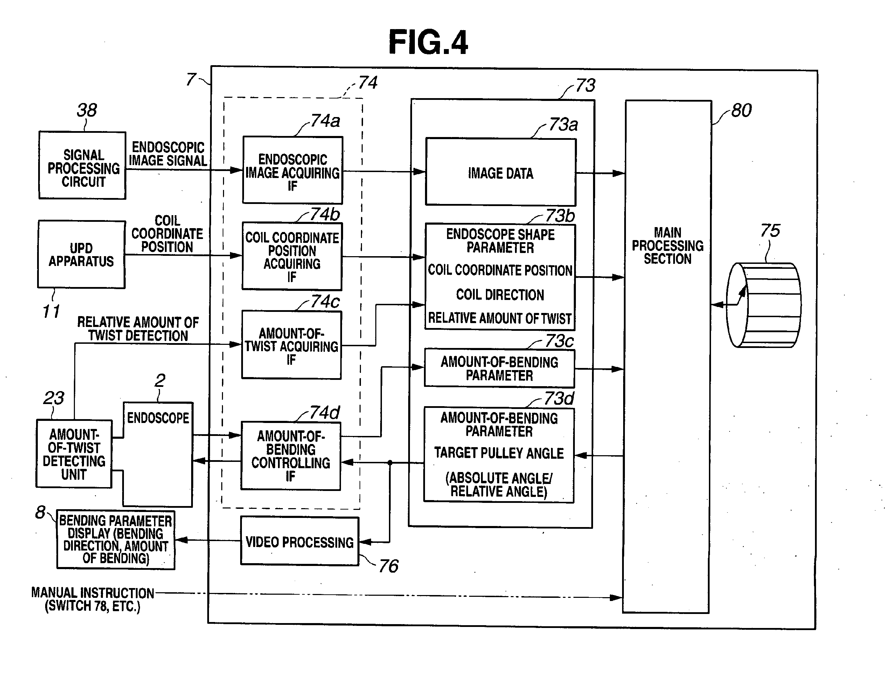

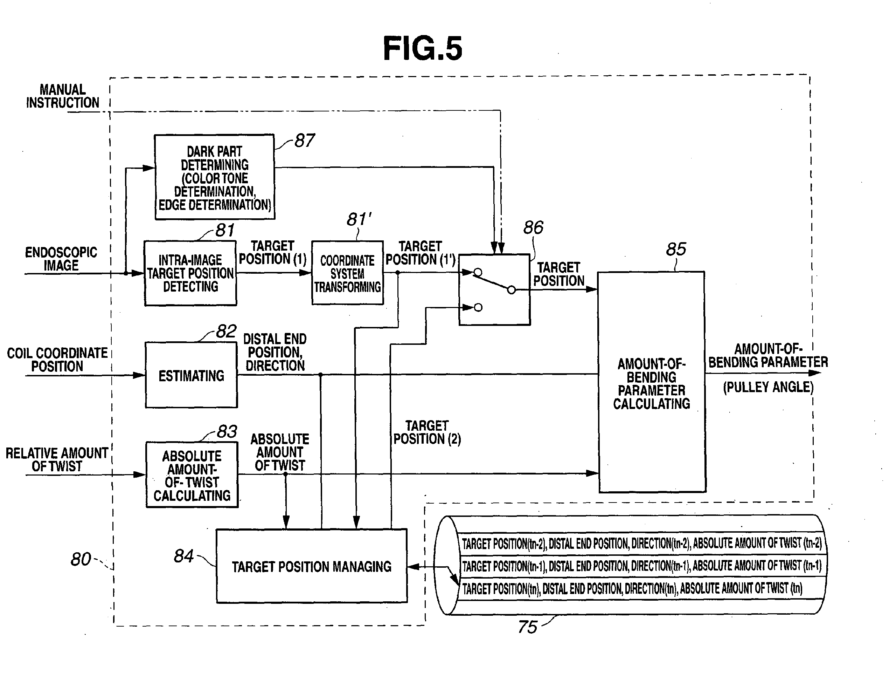

FIGS. 1 to 12 relate to the first embodiment of the present invention. FIG. 1 shows an overall configuration of an endoscope system according to the first embodiment of the present invention. FIG. 2 shows a specific configuration of FIG. 1, FIG. 3 shows a configuration of an amount-of-twist detecting unit. FIG. 4 shows a functional block of a PC main body, and FIG. 5 shows a functional configuration of bending control by a main processing section.

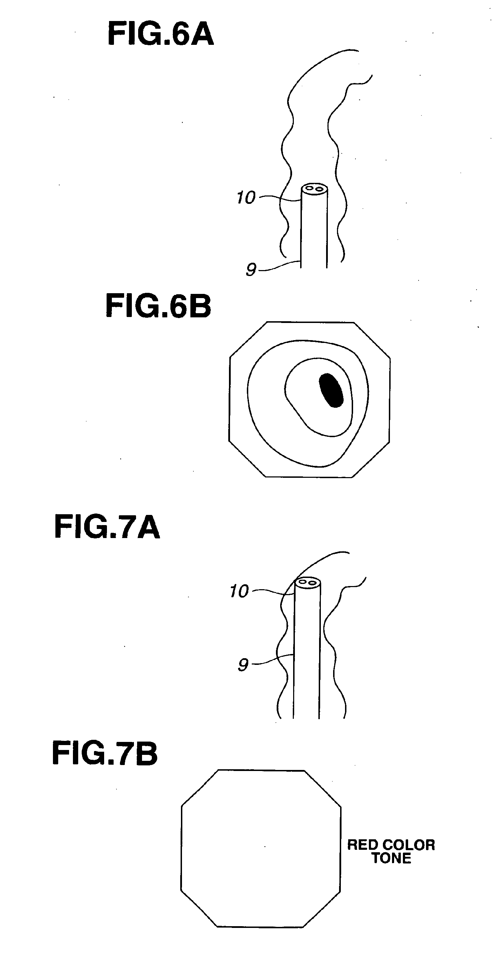

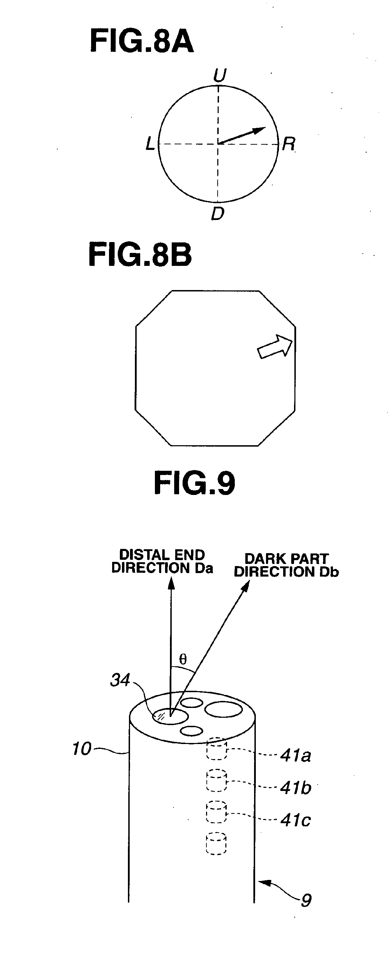

FIG. 6 shows a state where an insertion portion of an endoscope is inserted in a large intestine, and an exemplary image which can be acquired when a dark part exists in the image in the state. FIG. 7 shows a state where the insertion portion of the endoscope is inserted in the large intestine, and an exemplary image from which the dark part has disappeared in the state. FIG. 8 shows a display example in which a bending direction and the like are displayed.

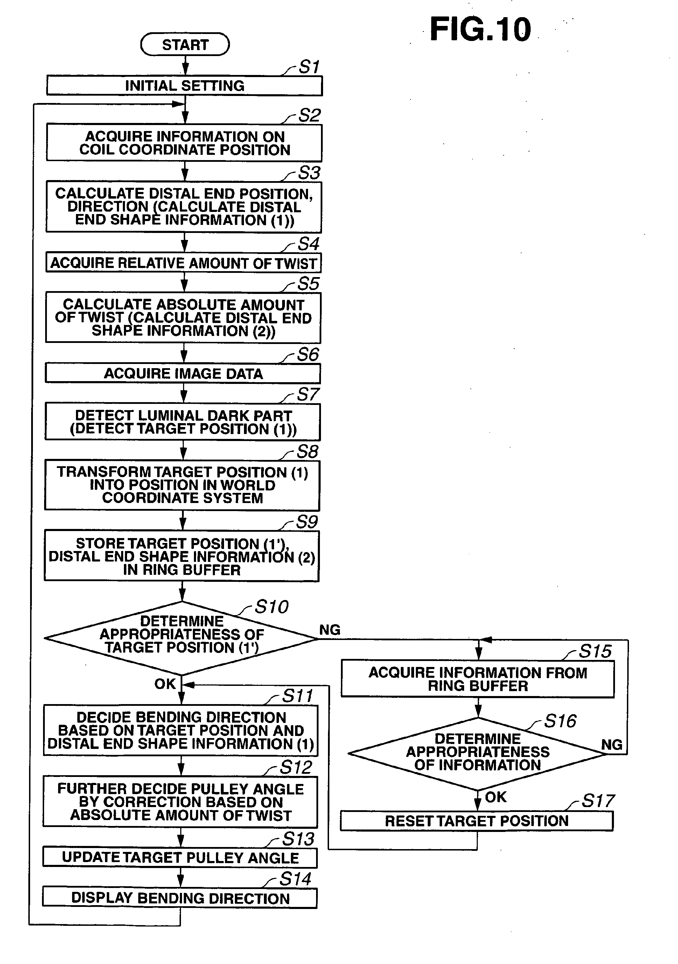

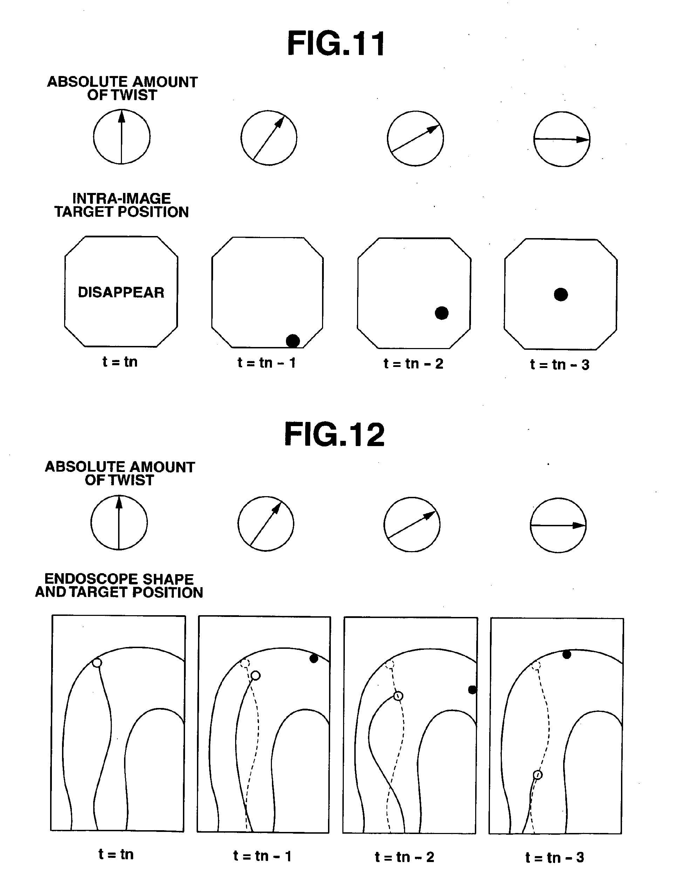

FIG. 9 shows an operation of bending control for bending a bending portion in a direction of the dark part, FIG. 10 shows an operation content of the main processing section of the present embodiment, FIG. 11 is an operation illustration diagram showing information on absolute amounts of twist and corresponding intra-image target positions which are stored in a ring buffer in order of time, and FIG. 12 shows information on the absolute amounts of twist and corresponding shapes of the endoscope which are stored in the ring buffer in order of time.

As shown in FIGS. 1 and 2, an endoscope system 1 according to the first embodiment of the present invention includes: an endoscope apparatus 6 including an endoscope 2 for performing endoscopic examination, a light source apparatus 3, a processor 4 and an endoscope monitor 5; a personal computer main body (hereinafter referred to shortly as PC main body) 7 as an image processing apparatus for performing image processing for bending control and the like on an endoscopic image picked up by the endoscope 2; a PC monitor 8; and a UPD (registered trademark in Japan and U.S.A. owned by Olympus corp. Hereinafter, only referred to as UPD.) apparatus 11 having a function as position detecting means that detects at least a distal end portion 10 of an insertion portion 9 of the endoscope 2.

As shown in FIG. 1, the endoscope 2 includes the elongated insertion portion 9 to be inserted in the body cavity of a patient 13 lying on a bed 12, and an operation portion 14 provided at a rear end of the insertion portion. A connector located on an end portion of a universal cable 15 extended from the operation portion 14 is connected to the light source apparatus 3 for emitting illumination light and the processor 4 as a signal processing apparatus for performing signal processing.

As shown in FIG. 2, the insertion portion 9 includes a distal end portion 10 provided at the distal end thereof, a bendable bending portion 18, and a flexible portion 19 having flexibility and extended from a rear end of the bending portion 18 to the operation portion 14.

The operation portion 14 is provided with a joystick 21, for example, as bending instruction operation means that performs a bending instruction operation to bend the bending portion 18 in a direction desired by a surgeon 20. The surgeon 20 operates the joystick 21, thereby capable of electrically bending the bending portion 18 through a motor unit 22 as an electric bending driving means provided in the operation portion 14.

Furthermore, in the present embodiment, an amount-of-twist detecting unit 23 is provided on a rear-side outer circumferential surface of the insertion portion 9, for example, so as to be able to detect the amount of twist when the insertion portion 9 is twisted (wrenched) around the axis thereof.

As shown in FIG. 2, a light guide 31 for transmitting illumination light is inserted through the insertion portion 9 and the rear end of the light guide is connected, via the operation portion 14 and the universal cable 15, to the light source apparatus 3. On the rear end surface of the light guide 31 is incident illumination light from a lamp 32 in the light source apparatus 3. The illumination light transmitted by the light guide 31 comes out from a light guide distal end surface that is fixed to an illumination window provided in the distal end portion 10, and is emitted further forward through an illumination lens 33 opposed to the light guide distal end surface.

The illumination light emitted forward of a longitudinal axis of the distal end portion 10 from the illumination window illuminates forward of the longitudinal axis in the body cavity into which the insertion portion 9 is inserted. Then the illumination light illuminates an observation field of view of an objective lens 34 described below or an image pickup range.

The objective lens 34, which forms an optical image of the inside of a body cavity as an object to be observed, is mounted to an observation window (image pickup window) provided adjacent to the illumination window. An image pickup apparatus 36 is configured of the objective lens 34 and a CCD 35, for example, as a solid-state image pickup device arranged at the image-forming position of the objective lens.

The CCD 35 is connected to a CCD driving circuit 37 and a signal processing circuit 38 in the processor 4 through a signal line inserted through the insertion portion 9. The CCD driving circuit 37 generates a CCD driving signal to apply the generated signal to the CCD 35. Upon receiving the CCD driving signal, the CCD 35 photoelectrically converts the optical image formed on the image pickup surface of the CCD 35 and outputs the photoelectrically converted optical image as a CCD output signal or an image pickup signal.

The image pickup signal is inputted to the signal processing circuit 38. The signal processing circuit 38 performs signal processing on the image pickup signal and generates an RGB signal and the like, for example, as an endoscopic image signal (video signal) for displaying an endoscopic image on the endoscope monitor 5. The endoscopic image signal is inputted to the endoscope monitor 5 and the endoscopic image is displayed on an endoscopic image displaying area 5a of the endoscope monitor 5.

Note that the endoscopic image signal is inputted also to the PC main body 7 as an image processing apparatus and used for image processing for detecting position information to insert the distal end of the insertion portion 9 in the running direction of the body cavity. Furthermore, in the endoscope 2 according to the present embodiment, in order to detect the insertion shape (also referred to as endoscope shape) of the insertion portion 9, a plurality of coils (referred to as UPD coils) 41a, 41b, 41c, etc. as position information generating means, each of which generates position information, are arranged in the insertion portion 9 at predetermined intervals, for example, from a position in the distal end portion 10 to an appropriate position of the flexible portion 19.

By detecting the position of each of the UPD coils 41a, 41b, 41c, etc., the insertion shape of the insertion portion 9 can be calculated. By detecting the position of each of the plurality of UPD coils (for example, 41a, 41b and 41c) located on the distal end side of the insertion portion 9, in particular, in addition to the distal end position of the insertion portion 9, the longitudinal axis direction (orientation) of the insertion portion 9 can be detected.

Note that FIG. 2 shows an example in which the UPD coils are arranged in the insertion portion 9 of the endoscope 2. However, a probe in which the UPD coils 41a, 41b, 41c, etc. are provided may be inserted through a channel not shown, to detect the shape of the insertion portion through which the probe is inserted.

A cable on the rear end sides of the UPD coils 41a, 41b, 41c, etc. is connected to a UPD apparatus 11.

As shown in FIG. 2, the UPD apparatus 11 includes a UPD driving circuit 42 for driving the UPD coils 41a, 41b, 41c, etc. to cause the UPD coils to generate magnetic fields.

Furthermore, the UPD apparatus 11 includes a magnetic field detecting sense coil section 43 composed of a plurality of sense coils 43a, 43b, 43c, etc. which are arranged in a predetermined positional relationship to detect magnetic fields.

In addition, the UPD apparatus 11 includes: a UPD coil position detecting circuit 44 for detecting (calculating) the positions of the UPD coils 41a, 41b, 41c, etc. based on detection signals from the sense coils 43a, 43b, 43c, etc. which form the sense coil section 43; an insertion shape calculating/displaying processing circuit 45 that performs calculation processing of the insertion shape of the insertion portion 9 based on the position information of the UPD coils 41a, 41b, 41c, etc. and display processing of the calculated insertion shape; and a shape displaying monitor 46 that displays the insertion shape upon receiving the video signal generated by the display processing.

Note that at least the sense coil section 43 in the UPD apparatus 11 is arranged in the vicinity of the bed 12 in FIG. 1, and the sense coil section detects the positions of the UPD coils 41a, 41b, 41c, etc. in the coordinate system (referred to as the world coordinate system) which covers the three-dimensional region of the patient 13 lying on the bed 12, where the insertion portion 9 is inserted. In other words, the sense coil section detects the three-dimensional coordinate positions in the world coordinate system.

The endoscopic image acquired by the image pickup apparatus 36 provided in the distal end portion 10 changes according to an insertion amount of the insertion portion 9 in the body cavity (lumen such as large intestine in the description below).

Therefore, the position information of the dark part in the lumen (also referred to as luminal dark part) detected based on the endoscopic image is transformed into the world coordinate system. Note that the position information of the dark part corresponds to the running direction of the lumen, so that the position information shows the target position to which the distal end of the insertion portion is to be inserted (introduced) toward a deeper side of the lumen or a target position of the bending direction into which the distal end of the insertion potion is to be bent.

Note that the observation direction of the image pickup apparatus 36 provided in the distal end portion 10 is parallel to the longitudinal axis of the insertion portion 9 in the endoscope 2, and the insertion direction and the bending direction are the same as the observation direction of the image pickup apparatus 36.

Information on the coil coordinate positions of the UPD coils 41a, 41b, 41c, etc. which is detected, for example, by the UPD coil position detecting circuit 44 in the UPD apparatus 11 is also inputted to the PC main body 7.

As schematically shown in FIG. 2, the bending portion 18 is configured of a plurality of bending pieces rotatably connected to each other in the longitudinal direction. In addition, bending wires 51u, 51d, 51l and 51r are inserted through the insertion portion 9 along up-down and left-right directions. The rear ends of these bending wires 51u, 51d, 51l and 51r are connected to pulleys 52a, 52b configuring a motor unit 22 arranged in the operation portion 14, for example. (Note that FIG. 2 shows only the rear end sides of the bending wires 51l and 51r.)

In the operation portion 14 are disposed a pulley 52a on which a wire connected with the both ends of the up and down bending wires 51u, 51d is wound, and a pulley 52b on which a wire connected with the both ends of the left and right wires 51l, 51r is wound.

The pulleys 52a, 52b are connected to rotational axes of the motors 53a, 53b, respectively, and rotated according to the rotation direction of the motors 53a, 53b which are rotatable normally and reversely. The motors 53a, 53b are driven by a motor driving section 55, driving of which is controlled by the driving controlling section 54.

Thus a bending actuator, which electrically bends and drives the bending portion 18 through the bending wires 51u, 51d, 511 and 51r by rotating the pulleys 52a, 52b with the motors 53a, 53b, is configured.

Since the amount of bending of the bending portion 18 corresponds to the rotation amounts of the pulleys 52a, 52b rotated through the motors 53a, 53b, the rotation amounts of the pulleys 52a, 52b are called pulley angles.

The driving position of the bending actuator is detected by rotary encoders 56a, 56b as actuator position detecting means which are mounted to the rotational axes of the motors 53a, 53b, for example. The detection signals from the rotary encoders 56a, 56b are inputted to the motor driving section 55 and (passed through the motor driving section 55) to the driving controlling section 54, for example.

The amount of bending (bending angle) of the bending portion 18 can be detected based on the detection signals from the rotary encoders 56a, 56b.

The driving controlling section 54 controls the rotation drive amounts (corresponding to the pulley angles of the pulleys 52a, 52b) of the motors 53a, 53b through the motor driving section 55 based on the detection signals from the actuator position detecting means, thereby enabling the bending portion 18 to be bent to an instructed amount of bending.

That is, as described above, by using the joystick 21 as bending instruction operation means provided to the operation portion 14, an arbitrary bending direction of the up-down and left-right directions is instructed and command for the bending operation amount (bending angle) is issued.

By specifying the up-down and left-right directions and issuing the command for the bending operation amount, an up-down direction joystick motor 57a and a left-right direction joystick motor 57b are rotated. The rotation amounts of the joystick motors, that is, the bending operation amounts are detected by the rotary encoders 58a, 58b. The detection signals detected by the rotary encoders 58a, 58b are inputted to the driving controlling section 54.

The driving controlling section 54 controls the rotation drive amounts of the motors 53a, 53b through the motor driving section 55 such that the value of the rotation drive amounts coincide with that of the bending operation amount detected by the rotary encoders 58a, 58b.

Note that the rotation driving of the up-down direction joystick motor 57a and the left-right direction joystick motor 57b is controlled by the driving controlling section 54 which receives the detection signals from the rotary encoders 58a, 58b.

In addition, in the present embodiment, the driving controlling section 54 is connected to the PC main body 7 and is capable of performing bending control based on the bending control information (or bending information) from the PC main body 7.

The amount-of-twist detecting unit 23 that detects the amount of twist of the insertion portion 9 has a configuration as shown in FIG. 3, for example.

As shown in FIG. 3, the amount-of-twist detecting unit 23 includes, for example, a cylindrical-shaped housing 61, a pair of bearings 62, 63, which is arranged along a central axis of the housing, for rotatably holding the insertion portion 9, and a sensor 63 that detects the amount of twist of the insertion portion 9 (the sensor 63 is a generic name used to refer to the reference numerals 63a to 63h in FIG. 3).

The housing 61 includes a through hole through which the insertion portion 9 is passed. In the through hole are disposed the pair of bearings 62, 62 that rotatably supports the insertion portion 9. In addition, the housing 61 includes inside thereof a light emitting diode 63a (abbreviated as LED), a lens 63b, a slit disk 63c, a fixed slit 63d, photodiodes (abbreviated as PD) 63e, 63f, a comparison circuit 63g, and a counter 63h.

The LED 63a is fixed in the housing 61. The LED 63a emits light in the direction parallel to the axis of the housing 61, that is, the axial direction of the insertion portion 9. The lens 63b is disposed on the optical path of the LED 63a. The lens 63b collects incident lights to form a parallel luminous flux, for example.

The slit disk 63c which is mounted on the outer circumferential surface of the insertion portion 9 is disposed on the optical axis of the light which passes through the lens 63b.

The slit disk 63c includes a plurality of slits radially formed at a predetermined angle on the part on the end portion side in a circumferential direction. The fixed slit 63d is disposed on the rear side of the slit disk 63c.

The pair of PDs 63e, 63f is disposed on the rear side of the fixed slit 63d. Note that the fixed slit 63d has four slits provided substantially parallel to one another so that the four slits can transmit the lights which have transmitted through the four slits formed on the slit disk 63c, for example. The lights which have transmitted through the four slits are detected by the PD 63e.

Four more slits are provided adjacent to the four slits so as to oppose to a light shielding portion of the slit disk 63c. The lights which have transmitted through these four slits are detected by the PD 63f.

The detection signals from the PDs 63e, 63f are inputted to the comparison circuit 63g.

The comparison circuit 63g compares the detection signal from the PD 63e with a threshold based on the detection signal from the PD 63f. The comparison circuit 63g outputs H or a binary signal of 1 when the detection signal from the PD 63e is equal to or larger than the threshold, and outputs L or a binary signal of 0 when the detection signal is smaller than the threshold, for example.

The counter circuit 63h counts the output signal from the comparison circuit 63g to calculate a relative amount of twist of the insertion portion 9 shown by the outlined arrow in FIG. 3. Note that the relative amount of twist of the insertion portion 9 may be calculated based on only the detection signal from the PD 63e.

The relative amount of twist calculated by the counter circuit 63h is inputted to the PC main body 7. As shown in FIG. 2, the PC main body 7 includes: a CPU 71 that performs image processing for detecting a dark part as described later, and also performs image processing for bending control responding also to the case where the dark part has disappeared; a hard disk (abbreviated as HDD) 72, for example, for storing an image processing program and the like; a memory 73 used for temporal storage of data and as a work area; an interface section (abbreviated as IF section) 74 which serves as an interface for inputting endoscopic image signal and the like and outputting information on the control of amount of bending; and a ring buffer 75, for example as recording means which stores information that allows reproducing a past distal end state of the insertion portion 9.

The HDD 72 stores a program and the like of the processing performed by the CPU 71. The CPU 71 reads the program via an HDD IF 72a, thereby performing processing responding to the disappearance of the dark part, that is, the CPU 71 has a function as the main processing section 80 shown in FIG. 4.

In addition, as shown in FIG. 2, the bus, which is connected with the CPU 71, is connected with the PC monitor 8 through a video processing circuit 76 and is also connected with the keyboard 77 through a keyboard IF 77a.

The surgeon 20 can input data and perform various instructing operations to the CPU 71 through the keyboard 77. In addition, the surgeon 20 can give an instruction to manually activate the bending control responding to the case where the dark part has disappeared, through a switch 78 provided to the operation portion 14 of the endoscope 2, for example. Note that the switch 78 may be configured of a scope switch which is widely used as an instruction switch for the processor 4 and the like. Furthermore, the instruction can be given from the keyboard 77 and the like, instead of the switch 78.

As shown in FIG. 4, the endoscopic image signal outputted from the signal processing circuit 38 is stored, via an endoscopic image acquiring IF 74a (as an image inputting section) configuring an IF section 74, in an image data storing section 73a in the memory 73 which is data recording medium, for example, as image data of A/D converted endoscopic image. Note that the HDD 72 and a nonvolatile flash memory, not shown, and the like may be used instead of the memory 73.

In addition, information on the coil coordinate positions of the UPD coils 41a, 41b, 41c, etc. which is detected by the UPD apparatus 11 is stored, via a coil coordinate position acquiring IF 74b, in an endoscope shape parameter storing section 73b in the memory 73, as endoscope shape parameter, more specifically, data of a coil coordinate position, a coil direction (information on coil direction can be replaced with a plurality of coil coordinate positions). Note that the endoscope shape parameter mainly includes a parameter for distal end shape of the insertion portion 9, a parameter for the amount of twist of the insertion portion 9, and the like. Therefore, in the operation example (FIG. 10), description will be made using the distal end shape, the amount of twist, and the like.

The relative amount of twist detected by the amount-of-twist detecting unit 23 is stored, via the amount-of-twist acquiring IF section 74c, for example, in the endoscope shape parameter storing section 73b in the memory 73.

The amount-of-bending parameter of the motor unit 22 of the endoscope 2 from the driving controlling section 54 of the endoscope 2 is stored in a (first) amount-of-bending parameter storing section 73c in the memory 73, via an amount-of-bending controlling IF section 74d.

The main processing section 80 configured of the CPU 71 stores, at every set time, the above-described image data, the endoscope shape parameter, and the amount-of-bending parameter in the memory 73 synchronously with the set time.

The main processing section 80 performs the processing as shown in FIG. 5 on the image data, the endoscope shape parameter, the amount-of-bending parameter, and sequentially store processed data and parameters in the ring buffer 75. FIG. 5 shows a functional configuration in the main processing section 80.

As shown in FIG. 5, the main processing section 80 includes a function of an intra-image target position detecting section 81 as position detecting means that detects target position (1) as position information based on luminal information in the endoscopic image, a function of an estimating section 82 that calculates the distal end position and direction of the insertion portion 9 based on (a plurality of) coil coordinate positions, and a function of an absolute amount-of-twist calculating section 83 that calculates the absolute amount of twist from the relative amount of twist.

The intra-image target position detecting section 81 detects, as position information, a center position (or position of the center of gravity) of the dark part corresponding to the running direction of the lumen in the endoscopic image, from the endoscopic image.

In addition, in the detected position of the dark part from the endoscopic image, values such as pixel size of the CCD 35 and focal point distance are taken into consideration. Based on the position information of the dark part with respect to the distal end position of the insertion portion 9 at the time, the direction of the dark part is detected as an insertion direction of the distal end of the insertion portion. Furthermore, based on the two-dimensional position information of the dark part, a three-dimensional position further including a value in the depth direction of the dark part is calculated by the Shape From Shading method, for example. The three-dimensional position information represents the target position (1) to which the distal end of the insertion portion 9 is to be oriented and introduced.

Note that the target position (1) detected by the intra-image target position detecting section 81 is transformed into a target position (1') of the world coordinate system by a coordinate system transforming section 81'.

Information on the target position (1'), the distal end position and direction (of the insertion portion 9), and the absolute amount of twist are stored, via a target position managing section 84 that manages target position used for bending control, in the ring buffer 75 in order of time (in a time-sequential manner).

As shown in FIG. 5, target position (1') information, the distal end position and direction information, and the absolute amount of twist information are stored in the ring buffer 75 in order of time in association with one another.

In FIG. 5, if the target position (1') information, the distal end position and direction information, the amount of twist information which are detected (calculated) at the time tn are defined as the target position (tn), the distal end position and direction (tn), the absolute amount of twist (tn), these pieces of information are stored in a memory cell for storing the information detected at the time tn.

Similarly, the pieces of information detected at the time tn-1 before the time tn are stored in a memory cell for storing the information detected at the time tn-1, which is adjacent to the memory cell for storing the information detected at the time tn. Pieces of information detected at the time tn-2 and other times are similarly stored. Note that when the target position (1') is read out from the ring buffer 75, the one target position is described as the target position (2). In addition, since the ring buffer 75 is made of m-number of memory cells, for example, the information on the target position (t1) stored at the time t1 is updated by the information on the target position (tm+1) stored at the time tm+1. Other pieces of information are similarly updated.

In addition, the distal end position and direction and the absolute amount of twist of the insertion portion 9 are inputted to (direction calculating means which outputs information on the insertion direction, and more particularly to) an amount-of-bending parameter calculating section 85 as bending information calculating means. The target position (1') and the target position (2) read out from the ring buffer 75 are inputted to the amount-of-bending parameter calculating section 85 via a target position switching section 86. The amount-of-bending parameter calculating section 85 calculates the amount-of-bending parameter using the target position inputted via the target position switching section 86, and outputs the calculated amount-of-bending parameter to the (second) amount-of-bending parameter storing section 74d in the memory 73 in FIG. 4.

In this case, the amount-of-bending parameter calculating section 85 uses the absolute amount of twist calculated by the amount-of-twist calculating section 83 to eliminate an influence caused in the case where the insertion portion 9 has been twisted during a period from the current time to a time retroactive from the current time, thereby performing accurate calculation of the amount of bending including a bending direction.

Furthermore, the amount-of-bending parameter calculating section 85 refers to the information on the distal end position and direction of the insertion portion 9 estimated by the estimating section 82, thereby performing accurate calculation of the amount of bending.

In addition, as shown in FIG. 5, the main processing section 80 also performs determination processing whether or not the intra-image target position detecting section 81 detects a target position from an endoscopic image under the set condition, that is, the condition in which a dark part exists.

Specifically, the main processing section 80 has a function of a dark part determining section 87 that determines existence or nonexistence of a dark part from the endoscopic image, and performs a color tone determination, an edge determination (or gradient determination), for example, as specific processings for determining the existence or nonexistence of the dark part.

When determining the existence or nonexistence of the dark part based on the color tone determination, the dark part determining section 87 calculates the color tone mean value of entire RGB signals corresponding to the endoscopic image. When the color tone mean value becomes a value representing a red color tone which exceeds the threshold for determining the nonexistence of the dark part, the dark part determining section 87 determines that no dark part exists.

Alternatively, the determination may be made using an XYZ chromaticity coordinate, an R/G value, and the like which are calculated based on the RGB signals.

FIG. 6(A) shows an example of an insertion state in which a dark part is detected with the insertion portion 9 inserted in the large intestine. The endoscopic image acquired in this insertion state is as shown in FIG. 6(B), and the dark part is detected.

In contrast, FIG. 7 (A) shows an example of an insertion state in which no dark part is detected. The endoscopic image in this insertion state is as shown in FIG. 7(B), and no dark part is detected. In the insertion state, the entire endoscopic image becomes red color tone, so that the insertion state can be determined based on the color tone mean value. Note that the entire endoscopic image becomes red color tone as shown in FIG. 7(B), the image is called "red-ball state" image.

In addition, when determining the existence or nonexistence of the dark part, instead of using the color tone mean value of the entire endoscopic image, the determination may be made by calculating the edge or gradient of the endoscopic image using a known Sobel filter, for example. The Sobel filter is a filter for detecting an edge. The existence or nonexistence of the dark part may be determined based on a collected value of the gradient values of the entire endoscopic image at the time that the Sobel filter is applied.

When the dark part disappears, a proximate image is picked up with the distal end of the endoscope being approximately perpendicular to the mucosal surface in the lumen, so that the collected value of the gradient values becomes smaller (compared with the case where the dark part exists). Accordingly, by comparing whether or not the collected value of the gradient values is smaller than a certain threshold, the determination of the existence or nonexistence of the dark part can be made.

When the dark part determining section 87 determines that a dark part exists, information on the target position (1') is inputted to the amount-of-bending parameter calculating section 85, as shown in FIG. 5. On the other hand, when the dark part determining section 87 determines that no dark part exists, the target position switching section 86 is switched and information on the target position (2) corresponding to a time retroactive from the current time read out from the ring buffer 75 is inputted to the amount-of-bending parameter calculating section 85, via the target position managing section 84.

Note that, in this case, as the processing to be described later with reference to FIG. 10, the target position managing section 84 performs processing for determining whether or not the information on the target position (2) read out from the ring buffer 75 retroactively is appropriate for the target position to be used in the bending control. The target position managing section 84 controls (the selection of the target position (2) from the ring buffer 75) such that the appropriate target position is inputted to the amount-of-bending parameter calculating section 85.

As described above, when the existence of the dark part is determined in the image processing by the dark part determining section 87, the existence of the dark part is used as a condition in the operation of detecting the position information from the dark part in an image.

As in the case where the dark part disappears from the image as described above, when it is determined that the image does not satisfy the condition, the position information of the dark part is not detected in the image and past information in which the dark part exists is used. As a result, the detection accuracy of the position information can be ensured.

Furthermore, when the surgeon 20 manually gives an instruction for responding to the disappearance of the dark part by operating the switch 78, for example, the main processing section 80 reads out from the ring buffer 75 the information on the past target position (2) by going back from the current time, via the target position managing section 84.

Then the main processing section 80 calculates the amount-of-bending parameter (pulley angle) used for bending the distal end of the insertion portion 9 such that the current direction of the distal end of the insertion portion 9 is directed toward the past target position (2). The amount-of-bending parameter calculating section 85 in the main processing section 80 thus performs detection processing of the target position (1') in the world coordinate system and calculates an amount-of-bending parameter for orienting (directing) the distal end portion 10 toward the target position (1'). The amount-of-bending parameter is then stored in the amount-of-bending parameter storing section 74d in the memory 73 in FIG. 4.

The amount-of-bending parameter is a pulley angle as a rotation amount of the pulleys 52a, 52b with respect to the rotation amount of the motor 53a, 53b of the motor unit 22, that is, a target pulley angle for rotating the pulley 52a, 51b by a target rotation amount.

The target pulley angle may be detected as an absolute angle for bending the bending portion from a neutral state (non-bending state) to a target pulley angle, or as a relative angle for relatively bending the distal end portion of the insertion portion at the current time to a target pulley angle, for example.

The amount-of-bending parameter stored in the memory 73 is sent, as bending control information, to the driving controlling section 54 of the endoscope 2 via the amount-of-bending controlling IF 74d. Then, the amount-of-bending parameter is used for bending control.

The driving controlling section 54 rotates the motors 53a, 53b of the motor unit 22 to bring the pulley angle into a state of the target pulley angle.

In addition, the amount-of-bending parameter is outputted to the PC monitor 8 via the video processing circuit 76, for example, and the bending direction and the amount of bending are displayed on the display screen of the PC monitor 8. The display example in this case is shown in FIG. 8(A).

In the display example in FIG. 8(A), on the display screen showing the up-down, and left-right bending directions (abbreviated as U, D, L and R) of the bending portion 18, the bending direction and the amount of bending in the case where the joystick 21 is bent so as to achieve the target pulley angle are shown by the arrow, for example. In this display example, the amount of bending is shown by the length of the arrow. However, the amount of bending may be displayed by numeric values.

Since the motor unit 22 is provided in the present embodiment, description will be made taking the case where the joystick 21 is also driven as an example. However, in the case of manual bending (to be described later) where the motor unit 22 is not provided, a bending operation direction in which a bending operation knob is to be operated and the amount of bending operation by manual operation may be displayed on the PC monitor 8 as display means.

Note that the display example is not limited to one in which the bending information such as the bending direction and amount of bending is displayed on the display screen of the PC monitor 8. The amount-of-bending parameter may be outputted to the processor 4, for example, and displayed on the endoscope monitor 5. The display example in this case is shown in FIG. 8 (B). In the display example in FIG. 8 (B), the bending direction and the amount of bending are displayed in the endoscopic image, for example. Note that only the bending direction may be displayed. In addition, the bending direction and the like may be displayed outside the endoscopic image.

As described above, the driving controlling section 54, based on the amount-of-bending parameter sent via the amount-of-bending controlling IF 74d, rotates and drives the motors 53a, 53b so as to achieve the parameter, and drives the pulley 52a, 52b so as to reach the target pulley angle.

As a result, the bending portion 18 is bent, and the distal end of the insertion portion 9 is controlled to be bent as shown in FIG. 9, for example. The distal end of the insertion portion 9 is controlled to be bent such that a direction Da of the distal end of the insertion portion 9 estimated by the main processing section 80 coincides with a direction Db of the calculated dark part (target position corresponding to the running of the lumen). In the case shown in FIG. 9, bending control is performed such that an angle θ is formed between the two directions.

In other words, in the present embodiment, the directions Da, Db are detected and bending control of the motor unit 22 as an electric bending driving mechanism is performed so as to render the distal end direction Da coincide with the dark part direction Db.

The bending control is thus performed such that the distal end of the insertion portion 9 is directed to the direction Db of the dark part, thereby enabling the surgeon 20 to smoothly insert the insertion portion 9 toward a deep part of the body cavity by push-in operation of the insertion portion 9, for example.

Furthermore, as described above, the main processing section 80 can perform control processing of the bending direction in response to a manual instruction by the surgeon 20.

In this case, the main processing section switches the target position switching section 86 in response to the manual instruction by the surgeon, as shown in FIG. 5. That is, similarly in the case where the target position switching section 86 is switched in response to the signal representing the determination of nonexistence of the dark part by the image processing, the target position switching section 86 can be switched in response to the instruction signal for instructing the nonexistence of the dark part by manual instruction.

Thus, in the present embodiment, the bending control can be performed by determining the existence or nonexistence of the dark part by the image processing. Moreover, even when the dark part disappears, the bending control can be performed such that the bending portion 18 is directed in the running direction of the lumen by the manual instruction of the surgeon 20.

Next, a content of the processings performed by the main processing section 80 according to the present embodiment will be described with reference to FIG 10. In FIG. 10, description is made on the case where the bending control is automatically performed based on the result of the image processing.

When the operation starts, the initial setting processing in step S1 is performed. In the initial setting processing, the main processing section 80 performs processing such as clearing of the memory content of the ring buffer 75, setting of the time interval to be stored in the ring buffer 75.

In the next step S2, the main processing section 80 acquires information on the coil coordinate positions of the UPD coils 41a, 41b, 41c, etc. which are detected by the UPD coil apparatus 11. In step S3, the estimating section 82 in the main processing section 80 in FIG. 5 calculates the current distal end position and direction of the insertion portion 9 based on the information on the coil coordinate positions of the UPD coils 41a, 41b, 41c, etc.. The distal end shape information (posture information) indicating the distal end position and direction in this case is also shown as the distal end shape information (1).

In the next step S4, the main processing section 80 acquires a relative amount of twist. Then, in the next step S5, the absolute amount-of-twist calculating section 83 in the main processing section 80 calculates the current absolute amount of twist in the case where the relative amount of twist as an initial value is zero, for example.

Based on the absolute amount of twist, the distal end position and direction are calculated by correcting the distal end shape information (1) indicating the distal end position and direction. The distal end shape information in this case is referred to as the distal end shape information (2) (even if a twisting operation was performed before the time when the information is obtained, the distal end shape information (2) is the information on the absolute position and direction of the distal end, which is not influenced by the twisting operation).

In the next step S6, the main processing section 80 acquires the image data of an endoscopic image. In step S7, the intra-image target position detecting section 81 in the main processing section 80 detects the luminal dark part, and detects the target position (1) to direct the distal end of the insertion portion 9 (by bending of the bending portion 18) in the direction of the dark part.

In the next step S8, the coordinate system transforming section 81' in the main processing section 80 transforms the target position (1) into a three-dimensional position in the world coordinate system used when the coil coordinate positions of the UPD coils 41a, 41b, 41c, etc. are calculated.

In the next step S9, the main processing section 80 stores the target position (1') in the world coordinate system and the distal end shape information (1) in the ring buffer 75. These pieces of information stored in the ring buffer 75 are shown in FIG. 5. Note that the distal end shape information (1) is, if the time when the distal end shape information (1) was obtained is tn, equivalent to the distal end position and direction (tn) and the absolute amount of twist (tn) in the example shown in FIG. 5.

In the next step S10, the main processing section 80 determines the appropriateness of the target position (1'). In this case, the dark part determining section 87 in the main processing section 80 determines the existence or nonexistence of the dark part based on the color tone and the like of the endoscopic image.

In this case, when the dark part exists, the main processing section determines that the target position (1') satisfies a predetermined accuracy, that is, the target position (1') is appropriate (OK). When it is determined that no dark part exists, the main processing section determines that the target position (1') is not appropriate (NG). When it is determined that the target position (1') is appropriate, the procedure moves on to the next step S11.

In the step S 11, the main processing section 80 decides the bending direction based on the current target position (1') and the distal end shape information (1), for example. Furthermore, in step S12, the main processing section 80 decides the pulley angle based on the distal end shape information (2) (that is, the current absolute amount of twist in the case where the initial value is set as zero). Note that the step S11 and the step S12 are combined and performed as one processing.

In the next step S 13, the main processing section 80 updates the target pulley angle by the decided pulley angle.

Furthermore, in step S 14, the information on the target pulley angle or the bending direction and the like as shown in FIG. 8 is displayed.

After that, the procedure returns to the step S2, the same processings are repeated on the coil coordinate position, the amount of twist, and the image data which are acquired at the next current time.

On the other hand, when it has been determined that the target position (1') is not appropriate in the step S10, the procedure moves on to step S 15. In the step S 15, the main processing section 80 acquires the target position (2) and the distal end shape information (2) from the ring buffer 75.

In the next step S16, the target position managing section 84 determines the appropriateness of the information on the target position (2) and the distal end shape information (2) acquired from the ring buffer 75. In other words, determination is made whether or not the target position (2) appropriately includes the dark part and satisfies the accuracy and the condition available as the target position for the bending control.

When the target position managing section 84 determines that the target position (2) cannot be used as a target position, the more previous information, which was acquired at the further previous time, than the information read out at the previous time (past time closest to the current time) is acquired from the ring buffer 75. Then, similarly, the target position managing section 84 determines the appropriateness of the information on the target position (2).

When it is determined that the target position (2) can be used as a target position, the target position (2) is reset as a target position in step S 17. After the resetting, the procedure returns to step S11. Then, based on the target position, bending control is performed.

Note that when the main processing section 80 is operated by manual instruction, the determination of the appropriateness of the target position (1') in step S10 in FIG. 10 is performed according to the manual instruction by the surgeon 20. When the manual instruction is not given, the procedure proceeds to the step S 11. On the other hand, when the surgeon 20 manually instructs that the dark part has disappeared, the procedure moves on to the step S 15. FIGS. 11 and 12 are operation illustration diagrams in the case where the main processing section 80 is operated by manual instruction.

FIG. 11 shows simple overview of the absolute amounts of twist calculated at the time tn, tn-1, tn-2, and tn-3 by the absolute amount-of-twist calculating section 83 and the intra-image target positions detected at the time tn, tn-1, tn-2, and tn-3, which are stored in the ring buffer 75.

FIG. 12 shows a simple overview of the absolute amount of twist calculated by the absolute amount-of-twist calculating section 83 shown in FIG. 11 and the endoscope shapes and the target positions. At the time tn-3, the intra-image target position is detected near the center of the endoscopic image.

After that, if the surgeon just pushes the rear end side of the insertion portion 9 in order to insert the distal end side of the insertion portion 9 toward the deep part in the lumen, at the next time tn-2 and the time tn-1, the intra-image target positions move from near the center to the edge of the endoscopic images.

If the surgeon further pushes the insertion portion 9 into the deep part of the lumen, the intra-image target position disappears at the time tn. In this state, the surgeon 20 operates the switch 78 and the like, to give manual instruction indicating the disappearance of the dark part to the main processing section 80, the main processing section 80 reads out the information on the target position at the time tn-1 or at the time tn-2 from the ring buffer 75, and calculates the bending direction in which the bending portion 18 is to be bent.

Then, the bending control may be performed through a bending controlling section 54. Alternatively, by displaying the bending direction and the like on the PC monitor 8, the surgeon 20 may bend the joystick 21 in the displayed bending direction.

Since the absolute amount of twist of the insertion portion 9 at past time is thus detected and stored also in the operation mode by manual instruction, even when the insertion portion 9 is twisted during the operation, the image can be accurately returned to the state in which the dark part is detected.

Thus, according to the present embodiment, when the insertion portion 9 is inserted into a body cavity such as the large intestine, the dark part is detected from the endoscopic image acquired by the image pickup means provided at the distal end of the insertion portion 9, and the bending portion 18 is controlled to be bent such that the distal end of the insertion portion 9 is directed in the direction in which the dark part is detected. Accordingly, the insertion portion 9 can be smoothly inserted into the deep part in the body cavity. In addition, the surgeon 20 can smoothly perform endoscopic examination.

With the PC main body 7 as an image processing apparatus according to the present embodiment, by connecting the PC main body 7 to the endoscope apparatus 6 and loading endoscopic images and the like, detection of the direction in which the distal end of the insertion portion 9 is inserted into the deep part in the body cavity and the bending control can be performed based on the image processing for detecting the dark part performed on the endoscopic image.

Note that the PC main body 7 exhibits substantially the same effects as described above also in the following first to fourth modified examples.

### (First Modified Example)

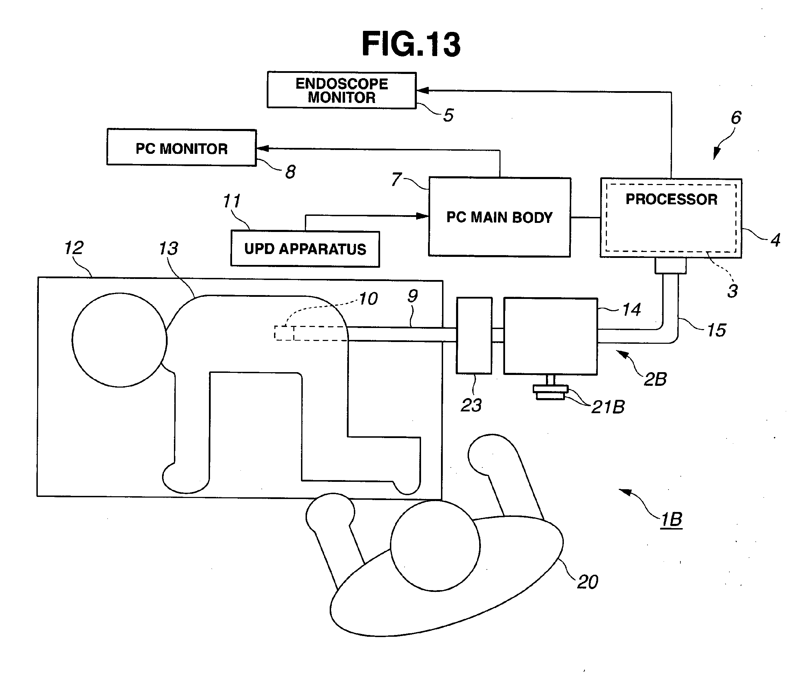

Next, the first modified example of the first embodiment will be described. FIG. 13 shows a configuration of an endoscope system 1B according to the first modified example.

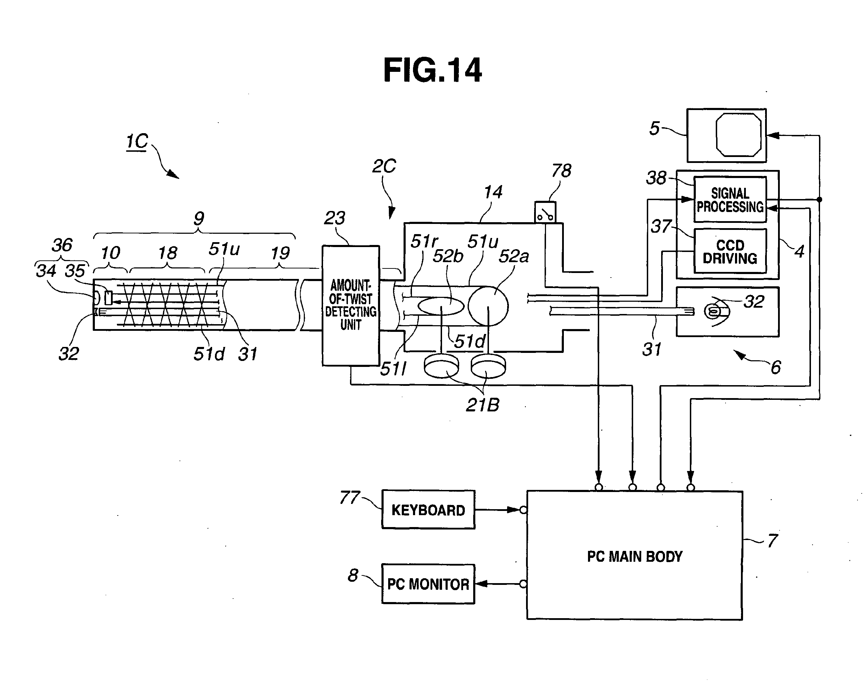

The first modified example shows the endoscope system 1 B having a configuration in which the motor unit 22 is eliminated from the endoscope system according to the first embodiment. Accordingly, an endoscope 2B according to the first modified example is configured by providing a bending operation knob 21 B connected to the rotational axes of the pulley 52a, 52b shown in FIG 2 in the operation portion 14 of the endoscope 2 in FIG. 1 (the configuration of this part is more specifically shown in FIG. 14 to be described later). The surgeon 20 rotates the bending operation knob 21 B, thereby capable of bending the bending portion 18 in arbitrary direction of up-down and left-right directions.

In the first modified example, the motor unit 22 is not provided, so that a processing for electrically driving and controlling the motor unit 22 performed in the first embodiment is not performed. In addition, in the first modified example, the information on the bending control by the PC main body 7, that is, the main processing section 80 is not outputted to the endoscope 2B which is manually bent. Information on the bending control is outputted to the PC monitor 8 or (via the signal processing circuit 38 as needed) to the endoscope monitor 5.

Then, on the PC monitor 8 or the endoscope monitor 5, the direction in which the bending operation knob 21B is to be bent, amount of bending, and the like are displayed (only the bending direction may be displayed). The display example in this case is the same as one shown in the above-described FIG. 8. However, in the present modified example, the direction in which the bending operation knob 21B is to be bent and the amount of bending are displayed.

Also in the present modified example, the dark part is detected from the endoscopic image, and the direction in which the bending operation knob 21 B is to be bent and the amount of bending are displayed. Accordingly, the surgeon 20 bends the bending operation knob 21 B as displayed, thereby capable of smoothly inserting (introducing) the insertion portion 9 into the deep part in the body cavity.

In addition, the present modified example can be widely applied to the endoscope 2B which is not provided with the motor unit 22.

### (Second Modified Example)

Next, the second modified example of the first embodiment will be described. FIG. 14 shows a configuration of an endoscope system 1C according to a second modified example.

The second modified example shows a configuration in which the UPD apparatus 11 is eliminated from the endoscope system 1B of the first modified example. In addition, the endoscope 2C according to the second modified example has a configuration in which the UPD coils 41a, 41b, 41c, etc. are eliminated from the insertion portion 9 in the endoscope 2B according to the first modified example.

The PC main body 7 has the same configuration as that in the first modified example. Note that in the case shown in FIG. 14, the PC main body 7 outputs the information on the bending control not only to the PC monitor 8 but also to the signal processing circuit 38 of the endoscope apparatus 6, thereby allowing the information on the bending control to be displayed both on the PC monitor 8 and the endoscope monitor 5. Note that the information on the bending control in this case can be displayed as shown in FIG 8, for example, similarly as in the case of the first modified example.

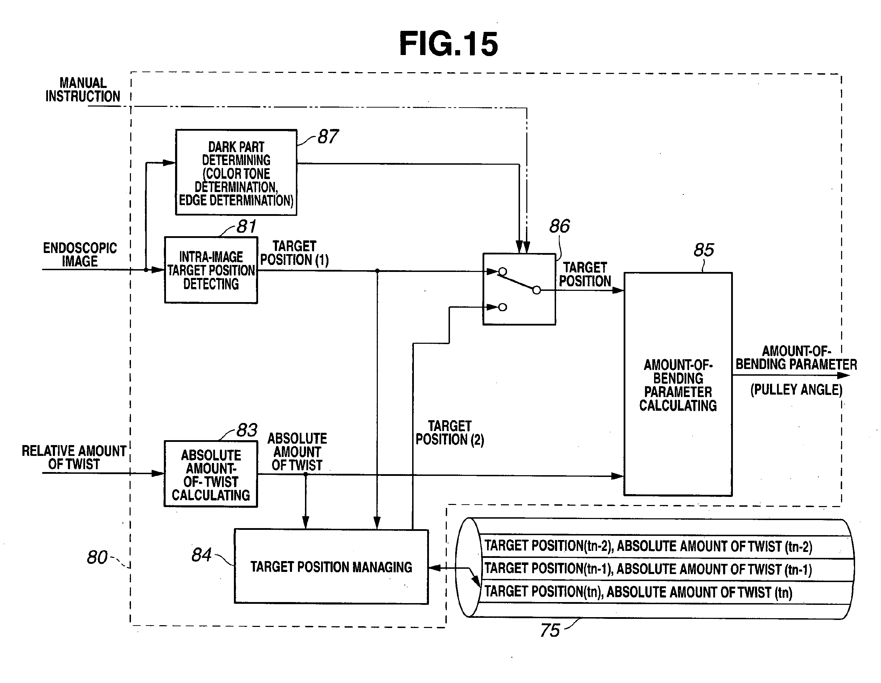

In addition, in the present modified example, the detection of the coil coordinate positions by the UPD coils 41a, 41b, 41c, etc. are not performed. Accordingly, a main processing section 80C included in the PC main body 7 has processing functions shown in FIG. 15, for example.

The processing functions shown in FIG. 15 do not include the functions of the estimating section 82 and the coordinate system transforming section 81' shown in FIG. 5. Furthermore, as described above, the information on the bending control, i.e., the amount-of-bending parameter calculated by the amount-of-bending parameter calculating section 85 in FIG. 15 is outputted to the PC monitor 8 and the signal processing circuit 38.

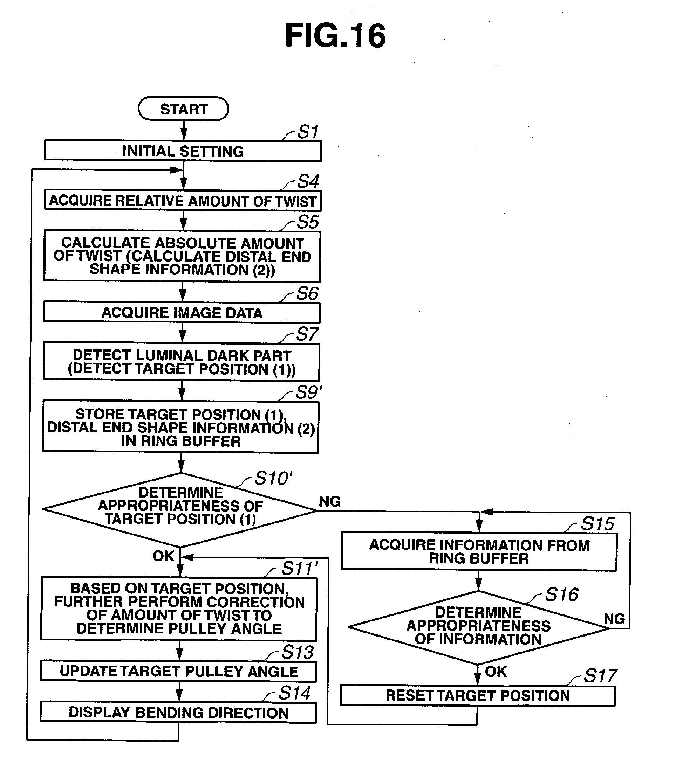

The processing procedure performed by the main processing section 80C in the present modified example is shown in FIG. 16. In the processing procedure shown in FIG. 16, some processings are omitted from the processing procedure shown in FIG. 10. Specifically, the above-described detection of the coil coordinate position using the UPD coils 41a, 41b, 41c, etc. is omitted from the procedure in FIG. 10. In addition, the transforming processing into the world coordinate system is also omitted. The processing content in FIG. 16 is described with reference to the processings in FIG. 10.

Similarly in the procedure in FIG. 10, after the initial setting processing in the first step S 1, the processings in the step S2 and the step S3 are skipped and the relative amount of twist acquiring processing in step S4 is performed. Next, the processings from the absolute amount of twist calculation in the step S5 to the detection of the luminal dark part in the step S7 are performed similarly as in the procedure in FIG. 10.

After the step S7, the transformation processing into the world coordinate system in the step S8 in FIG. 10 is skipped, and the target position (1) and the distal end shape information (2) are stored in the ring buffer in step S9'. In this case, not the target position (1') in FIG. 10 but the target position (1) is stored.

In the next step S10', the appropriateness of the target position (1) is determined. When the appropriateness determination of the target position (1) is OK, in step S11', correction of the amount of twist is further performed (in other words, the distal end shape information (2) is used) based on the target position (1), and thereby the pulley angle is decided.

Then, the target pulley angle is updated by the pulley angle in the step S 13, and the bending direction is displayed in the step S 14, and thereafter the procedure returns to the step S4. Note that the pulley angle and the target pulley angle in this case correspond to the amount of bending and the bending direction of the bending operation knob, so that the pulley angle and the target pulley angle may be replaced with the amount of bending and the bending direction of the bending operation knob.

On the other hand, in step S10', if the appropriateness determination of the target position (1) is NG, the procedure moves on to the step S15. The processings from the information acquiring processing from the ring buffer in the step S 15 to the target position resetting processing in step S 17 are the same as those in FIG. 10, so that descriptions thereof will be omitted.

The present modified example can be applied to the endoscope 2C which is not provided with the UPD coils 41a, 41b, 41c, etc.. Even when the dark part disappears, by using the past information in which the dark part exists, the information used for the bending control to bend the bending portion in the direction in which the dark part exists is displayed. Accordingly, the surgeon 20 performs bending operation as shown by the information for bending control, thereby capable of smoothly inserting the insertion portion 9 into the deep part in the body cavity.

In addition, even when the endoscope apparatus including the endoscope 2C which is not provided with the UPD coils 41a, 41b, 41c, etc. is used, the present modified example can be fabricated by providing processing means configured by the PC main body 7. Furthermore, there is no need to provide the UPD apparatus 11, so that the endoscope system 1C which allows smooth insertion can be constructed with reduced cost.

### (Third Modified Example)

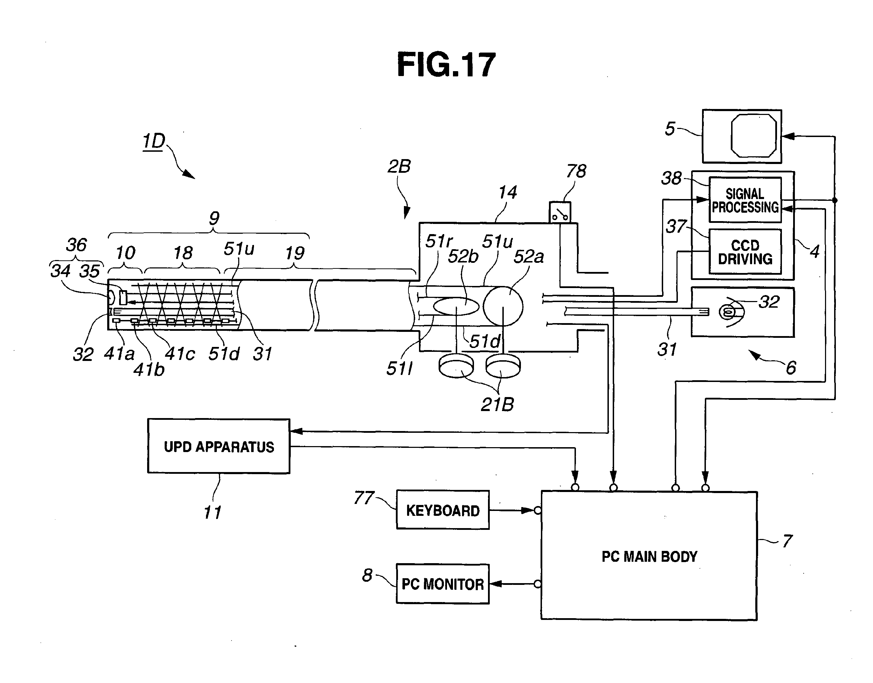

Next, the third modified example of the first embodiment will be described with reference to FIG. 17. The endoscope system 1D according to the third modified example shown in FIG. 17 has a configuration in which the amount-of-twist detecting unit 23 is eliminated from the endoscope system 1 B according to the first modified example.

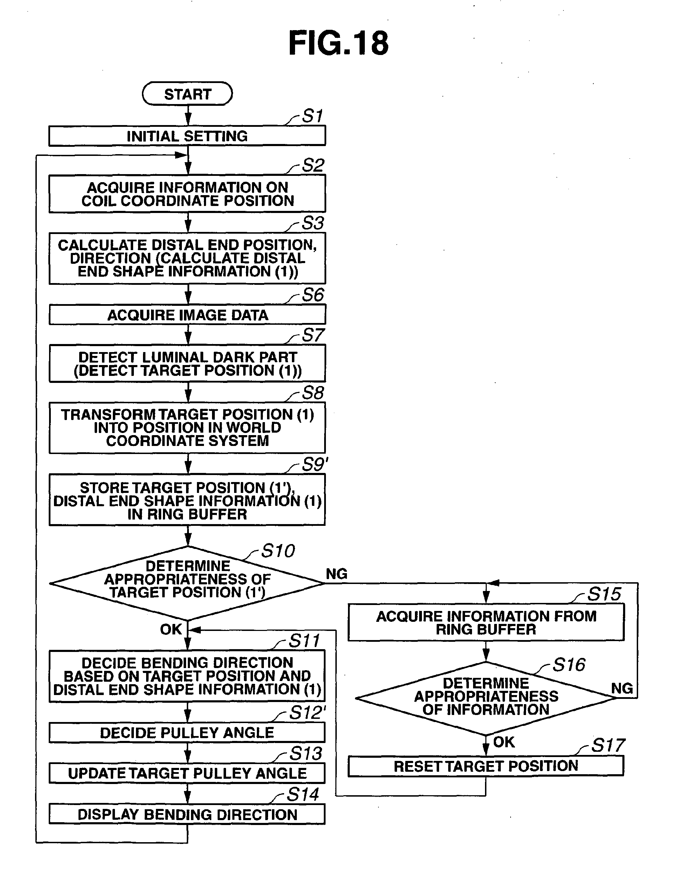

In the present modified example, the endoscope 2B in FIG. 13 showing the first modified example is used. However, in the present modified example, the amount-of-twist detecting unit 23 is not used. Therefore, in the present modified example, detection of the relative amount of twist by the amount-of-twist detecting unit 23 according to the first embodiment is not performed, for example. The processing procedure according to the present modified example is as shown in FIG. 18.

The processing procedure shown in FIG. 18 is basically the same as that in FIG. 10 but some processings are omitted. Therefore, description will be made with reference to the processing procedure in FIG. 10.

As shown in FIG. 18, the processings from the first step S1 to the step S3 are the same as those in FIG. 10. After the step S3, the steps S4 and S5 in FIG. 10 are skipped, and the image data acquiring processing in step S6 is performed. That is, processings of the calculation of the relative amount of twist by the amount-of-twist detecting unit 23 in step S4 and the calculation of the absolute amount of twist with respect to the relative amount of twist in step S5 are not performed.

After the step S6, the processings in steps S7 and S8 are performed similarly as in the procedure in FIG. 10.

Then, in the next step S9', the target position (1') and the distal end shape information (1) are stored in the ring buffer. In the present modified example, the distal end shape information (1) is used in place of the distal end shape information (2) in FIG. 10.

Then, similarly as in the procedure in FIG. 10, the appropriateness of the target position (1') is determined in the next step S10. When the target position (1') is appropriate, the processing in step S11 is performed similarly as in the procedure in FIG. 10. In the next step S12', the pulley angle is decided based on the result in step S11, and further in step S 13, the target pulley angle is updated. After the bending direction displaying processing in the next step S 14, the procedure returns to the step S2.

The processings in step S 15 and the subsequent steps, which are performed when the target position (1') is determined to be inappropriate in step S10, are performed similarly as in the procedure in FIG. 10.

In the present modified example, even when the dark part disappears, by reading out the information on the endoscopic image and the distal end position and direction before the dark part disappears, that is, in the state where the dark part exists, the direction in which the bending portion 18 is bent toward the target position corresponding to the dark part direction is detected to display the information on the direction.

As a result, also in the present modified example, the surgeon 20 can smoothly perform the insertion operation even in the state where the dark part is likely to disappear.

### (Fourth Modified Example)

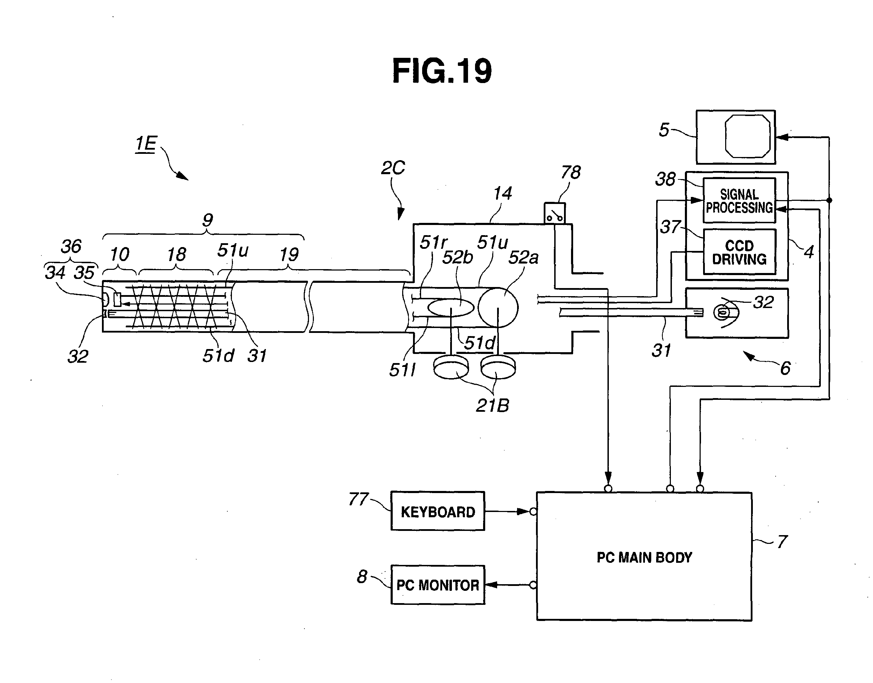

Next, the fourth modified example of the first embodiment will be described with reference to FIG. 19. An endoscope system 1E according to the fourth modified example shown in FIG. 19 is configured by using the endoscope 2D in which the UPD coils 41a, 41b, 41c, etc. are further eliminated, in the endoscope system 1D according to the third modified example.

In addition, since the UPD coils 41a, 41b, 41c, etc. are eliminated in the endoscope 2D, also the UPD apparatus 11 is eliminated.

To describe with reference to the endoscope system 1C in FIG. 14, the endoscope system according to the present modified example has the same configuration as that of the endoscope system 1C but the amount-of-twist detecting unit 23 is eliminated.

The processings in the present modified example are substantially the same as those in the above-described FIG. 18 but the processings in the steps S2, S3 and S8 are omitted. In addition, the target position (1) is used in the processings in FIG. 18, instead of the target position (1'). Other processings are the same as those in FIG. 18.

Also in the present modified example, when the dark part disappears, bending control information, which is used for bending the bending portion 18 in the direction of the dark part detected from the endoscopic image before the disappearance of the dark part, is displayed.

Therefore, also in the present modified example, the surgeon 20 can perform smooth insertion operation in the state where the dark part is likely to disappear.

Note that description has been made in the above-described first embodiment and modified examples thereof by taking as an example the case where the PC main body 7, which has a function as an image processing apparatus, displays the bending control information used for bending the bending portion 18 so as to direct the distal end of the insertion portion 9 in the running direction of the lumen or a body cavity based on (the luminal information) on the endoscopic image.

The information in this case can be read also as the information showing the direction in which the distal end of the insertion portion 9 is inserted (or moved) toward the running direction of the lumen or the body cavity. By reading the information in such a way, even when the bending portion 18 is not provided (for example, a capsule medical apparatus main body to be described in a second embodiment), the information can be applied as information used for inserting or moving the capsule in the running direction. Furthermore, in this case, the PC main body 7 includes a function as insertion portion distal end direction changing means that changes the direction of the distal end of the insertion portion.

When the capsule endoscope having image pickup means as a capsule medical apparatus main body is used, the above described first embodiment and modified examples thereof can be applied by regarding the end portion of the capsule-shaped insertion body on a side where the image pickup means is provided as the distal end of the insertion portion.

In the above-described first embodiment and modified examples thereof, description has been made on the image pickup system of the endoscope system 1 and the like in the case of using the endoscope 2 and the like which is to be inserted in a body cavity and which incorporates the image pickup means at the distal end of the insertion portion 9. In the second embodiment below, description will be made on a capsule medical system having a capsule medical apparatus main body which incorporates image pickup means in an insertion body to be inserted in a body cavity. Second Embodiment

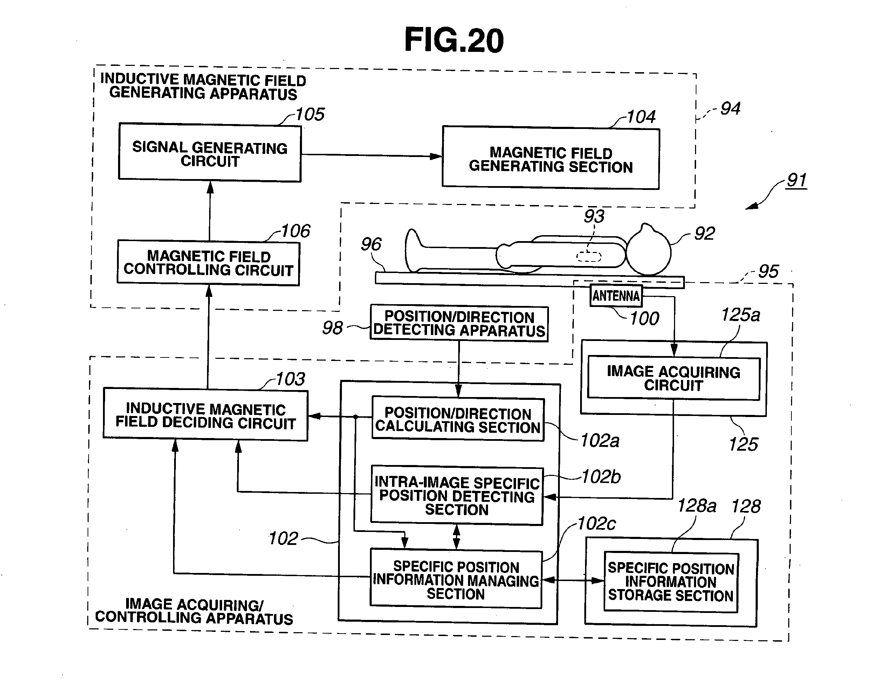

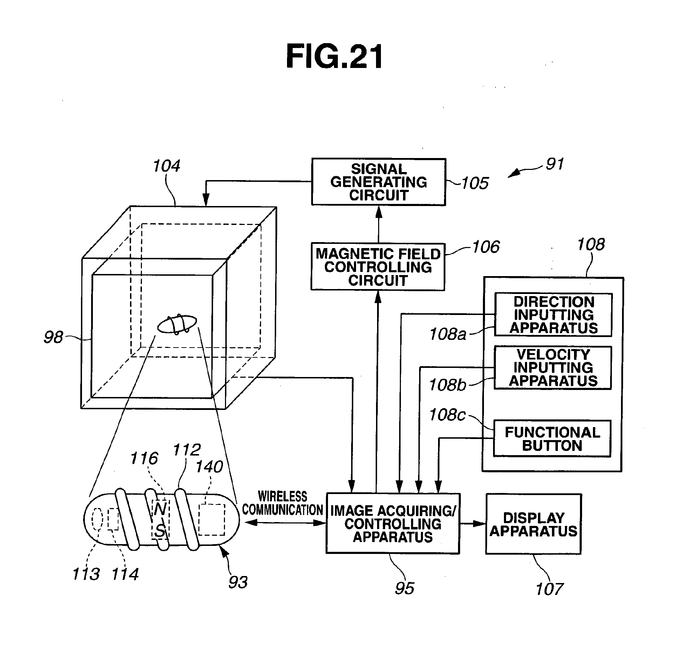

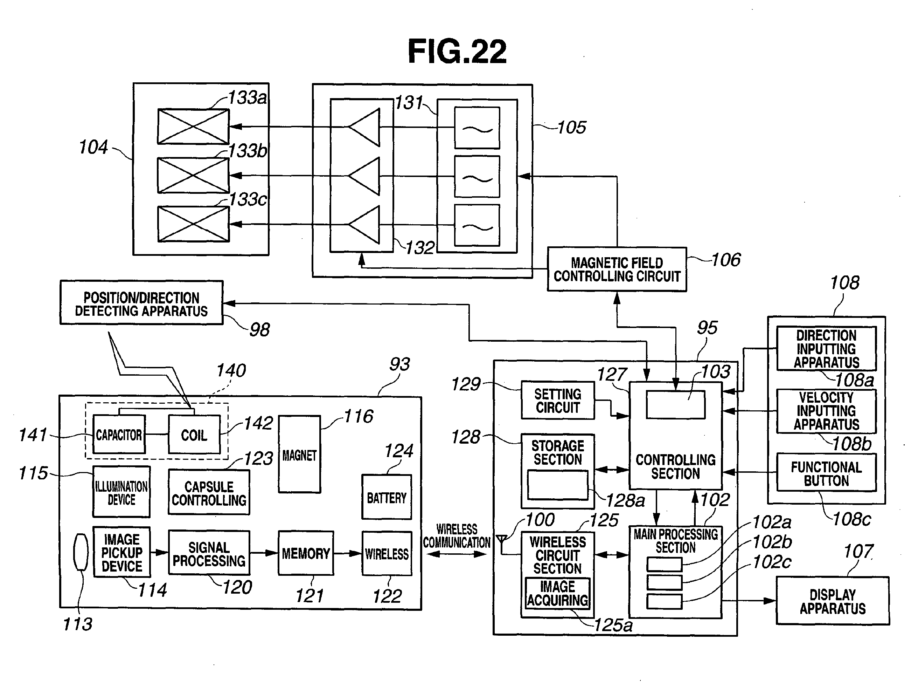

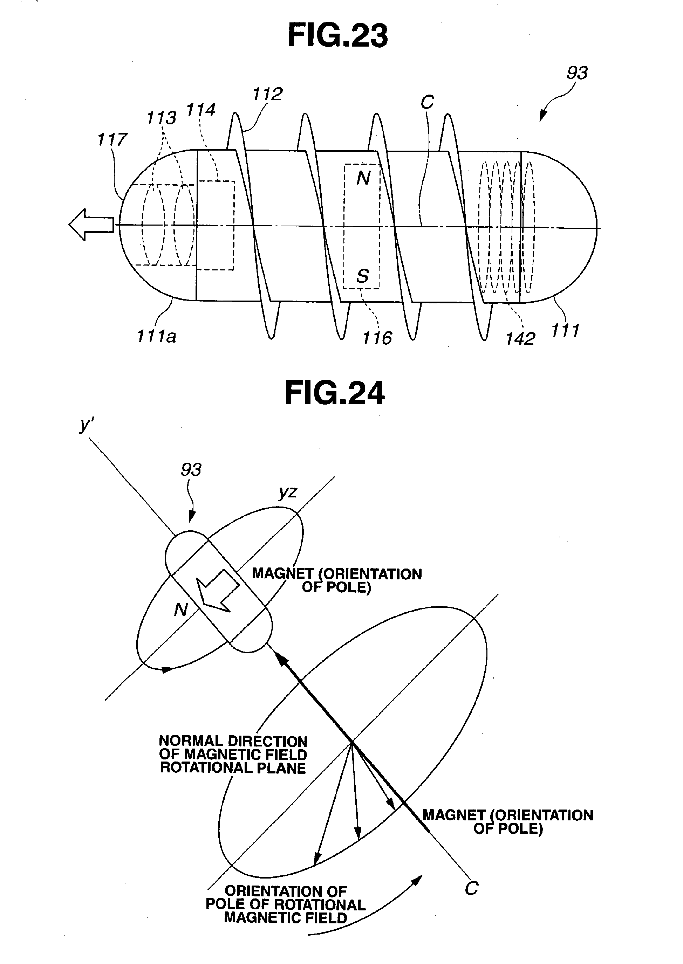

FIGS. 20 to 29 relate to the second embodiment of the present invention in which: FIG. 20 shows a configuration of a main part in the second embodiment of the present invention; FIG. 21 is an overall configurational view of a capsule medical system as an image pickup system according to the second embodiment; FIG. 22 is a more detailed block diagram of the capsule medical system in FIG. 21; FIG. 23 is an illustration diagram showing a side surface of a capsule main body; and FIG. 24 is a concept view showing an applied rotational magnetic field and how the capsule main body is operated by the rotational magnetic field.

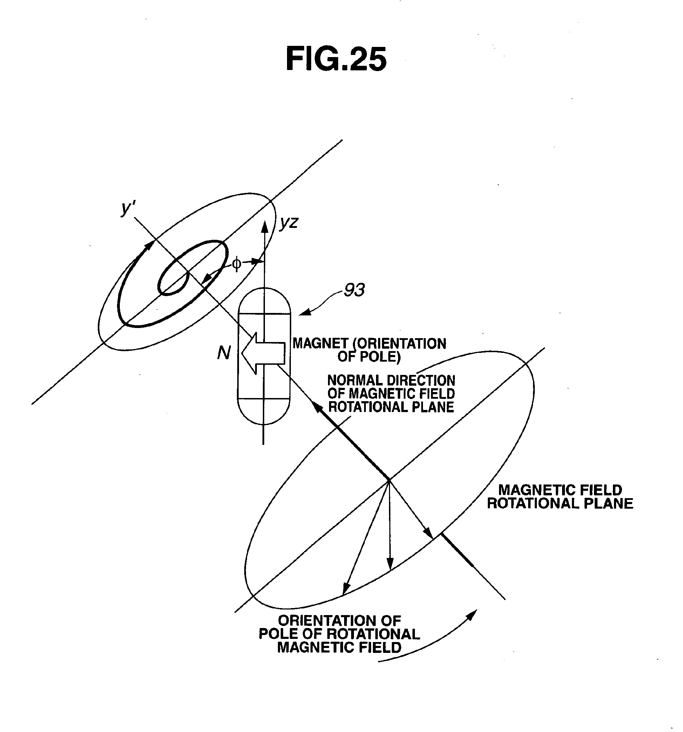

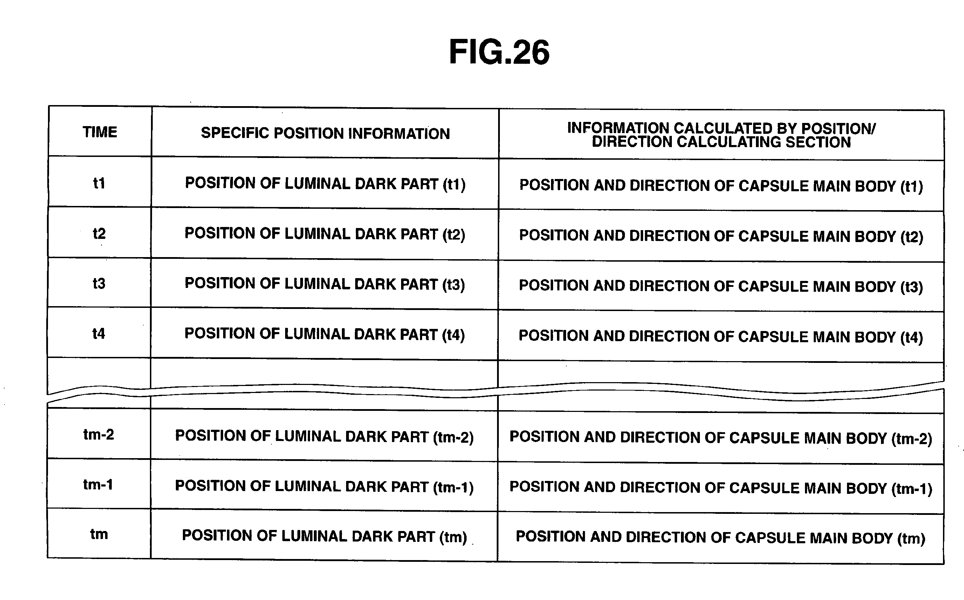

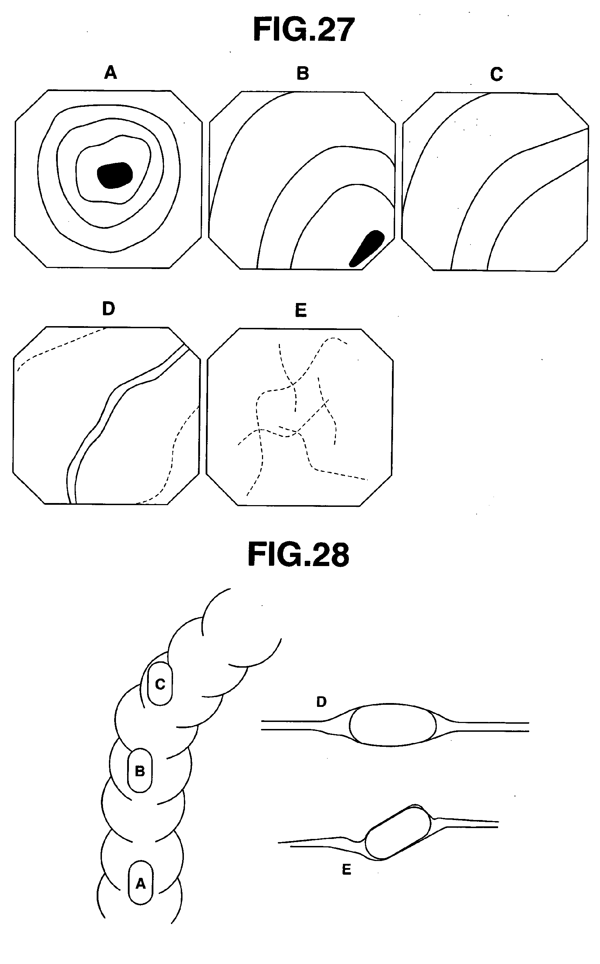

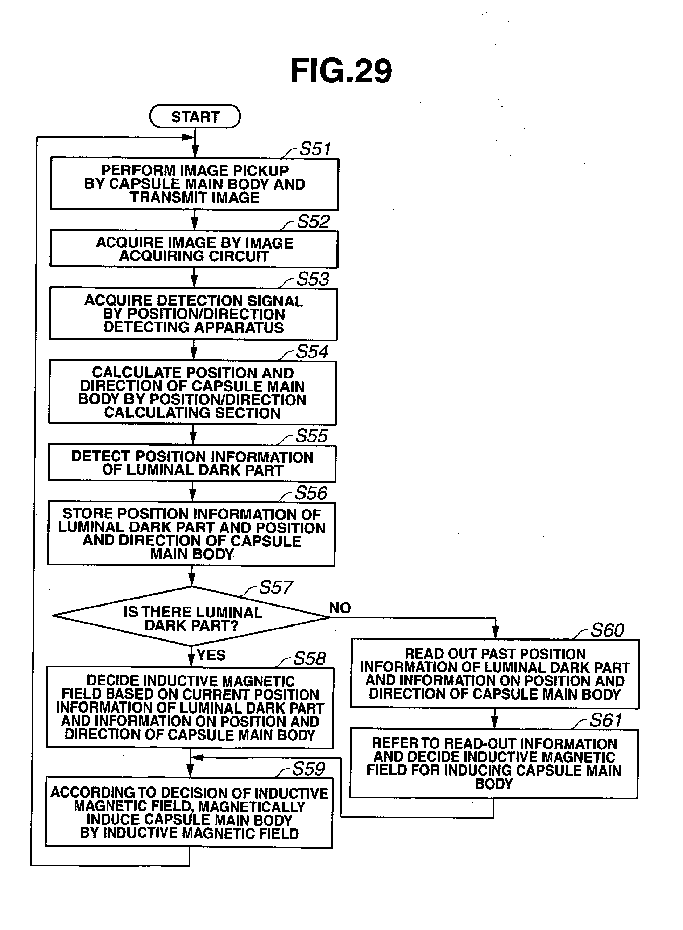

Furthermore, FIG 25 is a concept view showing a vibration magnetic field (couple generating magnetic field) applied to the rotational magnetic field in FIG. 24 and how the capsule main body is operated by the vibration magnetic field (couple generating magnetic field), FIG. 26 is a view showing specific position information and the like recorded in recording means in a time-sequential manner, FIG. 27 is a view showing examples of the images acquired by the image pickup means in the capsule main body, FIG. 28 is a view showing a capsule main body and a state of the lumen corresponding to each of the images in FIG. 27, and FIG. 29 shows an operation content of the second embodiment.

FIG. 20 shows a configuration of the main part of a capsule medical system 91 according to the second embodiment of the present invention. As shown in FIG. 20, the capsule medical system 91 according to the second embodiment of the present invention includes a capsule medical apparatus main body 93 (hereinafter referred to shortly as capsule main body) which is inserted into a body cavity of a patient 92 and serves as a capsule endoscope for picking up an image of the body cavity, and an inductive magnetic field generating apparatus 94 which is disposed around, that is, outside the body of the patient 92, and which applies a rotational magnetic field as the inductive magnetic field to the capsule main body 93 to induce the position and the longitudinal axis direction (orientation) of the capsule main body 93 from outside the body. Note that the capsule main body 93 is provided with the image pickup means in a predetermined direction as described later, so that the position and the direction of the image pickup means can be controlled by controlling the position and direction of the capsule main body 93 from outside the body. That is, such control enables the image pickup direction or the observation direction of the image pickup means to be controlled.

In addition, the capsule medical system 91 further includes an image acquiring/controlling apparatus 95 which is disposed outside the body of the patient 92, wirelessly communicates with the capsule main body 93, acquires the image picked up by the capsule main body 93, and controls the rotational magnetic field induced by the inductive magnetic field generating apparatus 94 by performing image processing on the acquired image.

The inductive magnetic field generating apparatus 94 includes: a magnetic field generating section 104 that generates a rotational magnetic field to be applied to the capsule main body 93 in the patient 92 lying on a bed 96; a signal generating circuit 105 that generates an alternating current signal used for causing the magnetic field generating section 104 to generate the rotational magnetic field; and a magnetic field controlling circuit 106 that controls the rotational magnetic field generated by the magnetic field generating section 104 by controlling the alternating current signal generated by the signal generating circuit 105.

Furthermore, the capsule medical system 91 includes a position/direction detecting apparatus 98 as a magnetic field detecting section that generates an alternating current magnetic field for causing a resonant circuit 140, which is to be described later and incorporated in the capsule main body 93, to generate induced electromotive force, and detects a magnetic field generated by the resonant circuit 140 which has generated induced electromotive force by the alternating current magnetic field, to detect the position and the longitudinal axis direction (orientation) of the capsule main body 93.

The detection signal detected by the position/direction detecting apparatus 98 is inputted to a position/direction calculating section 102a of the main processing section 102 in the image acquiring/controlling apparatus 95. The position/direction calculating section 102a calculates (estimates) the position and the direction of the capsule main body 93 based on the detection signal.

The information on the calculated position and direction of the capsule main body 93 is outputted to an inductive magnetic field deciding circuit 103 that decides the magnetic field controlling operation by the magnetic field controlling circuit 106, that is, the inductive magnetic field (more specifically, the rotational magnetic field) generated in the magnetic field generating section 104. Note that the position/direction detecting apparatus 98 and the position/direction calculating section 102a are integrally configured. In addition, the information on the calculated position and direction of the capsule main body 93 is displayed on a display apparatus 107 shown in FIG. 21 and the like.

In addition, the magnetic field controlling circuit 106 and the inductive magnetic field deciding circuit 103 may be integrally configured as an inductive magnetic field controlling circuit, for example. The processing of one of the circuits, which will be described below, may be performed by the integrally configured inductive magnetic field controlling circuit.

The image acquiring/controlling apparatus 95 receives a modulation signal including an image signal wirelessly transmitted from the capsule main body 93, by using an antenna 100, for example, which is mounted to the bed 96 and the like. The signal received by the antenna 100 is inputted to an image acquiring circuit 125a in a wireless circuit section 125, and the image acquiring circuit 125a demodulates the signal to generate an image signal (image data).

The image data is inputted to the intra-image specific position detecting section 102b as position detecting means or luminal information detecting means in the main processing section 102 configured by a PC, for example. The intra-image specific position detecting section 102b detects from the image data the position of the luminal dark part as the luminal information in the image, which is the intra-image specific position.

The position of the luminal dark part in the image corresponds to the running direction of the lumen, so that the direction of the position where the dark part is detected is regarded as a moving direction in which the capsule main body 93 is to be induced. Accordingly, the intra-image specific position detecting section 102b can serve also as estimating means which estimates the moving direction.

The information on the position of the luminal dark part is outputted to the inductive magnetic field deciding circuit 103 which decides the magnetic field controlling operation by the magnetic field controlling circuit 106. Based on the information inputted to the inductive magnetic field deciding circuit 103, the inductive magnetic field deciding circuit 103 decides, via the magnetic field controlling circuit 106, the intensity, the frequency and the like of the alternating current signal to be generated in the signal generating circuit 105. As a result, the rotational magnetic field to be generated in the magnetic field generating section 104 is also decided.

Note that the magnetic field controlling circuit 106 receives not only the information from the main processing section 102 shown in FIG. 20 via the inductive magnetic field deciding circuit 103 but also a signal for generating a magnetic field corresponding to an instruction signal in the case where an operator such as a surgeon manually gives an instruction, for example.

In addition, the information on the position of the luminal dark part detected by the intra-image specific position detecting section 102b is stored in a specific position information storage section 128a as recording means, via a specific position information managing section 102c. Note that the specific position information storage section 128a is set in a storage section 128 to be described later, for example, but not limited thereto.

The specific position information managing section 102c has a function as determining means which monitors or determines the detecting operation of the luminal dark part by the intra-image specific position detecting section 102b. For example, the specific position information managing section 102c acquires information on the existence or nonexistence of the luminal dark part, for example, as a condition set for the detecting operation of the position of the luminal dark part by the intra-image specific position detecting section 102b.

When the luminal dark part exists and the position thereof is detected, the specific position information managing section 102c stores the position information in the specific position information storage section 128a in order of time.

On the other hand, when the luminal dark part does not exist, the specific position information managing section 102c stops the information outputting operation from the intra-image specific position detecting section 102b to the inductive magnetic field deciding circuit 103. The specific position information managing section 102c refers to the specific position information stored in the specific position information storage section 128a, and, based on the information outputted from the specific position information managing section 102c, controls the decision of the inductive magnetic field for moving the capsule main body 93 by the inductive magnetic field deciding circuit 103.

Accordingly, the specific position information managing section 102c includes functions of means that detects the direction in which the capsule main body 93 is moved and of means that controls the movement of the capsule main body 93 via the inductive magnetic field deciding circuit 103 and the like.

When determining that the luminal dark part does not exist, the specific position information managing section 102c reads out the information acquired before the current time at which the luminal dark part is not detected, that is, the information acquired at a past time, as the specific position information stored in the specific position information storage section 128a, and performs control to generate an inductive magnetic field to bring the capsule main body 93 back into the state at the past time, for example.

Note that the specific position information managing section 102c shown in FIG. 20 determines the existence or nonexistence of the luminal dark part based on the information from the intra-image specific position detecting section 102b. However, the specific position information managing section 102c may determine the existence or nonexistence of the luminal dark part by directly loading the image data from the image acquiring circuit 125a.

In addition, a luminal dark part existence or nonexistence determining circuit may be provided to determine the existence or nonexistence of the luminal dark part from image data, and a position detecting circuit and the like may be provided to detect (calculate) the position of the luminal dark part based on the output signal of the luminal dark part existence or nonexistence determining circuit.

Note that the image acquiring/controlling apparatus 95 shown in FIG. 20 is connected with the display apparatus 107 and an operation inputting apparatus 108, as shown in FIGS. 21 and 22.

The image acquiring/controlling apparatus 95, which acquires the image picked up by the capsule main body 93 and controls the direction, the intensity and the like of the rotational magnetic field as the inductive magnetic field to be applied to the capsule main body 93, is connected with the display apparatus 107 which displays the image and the like picked up by the capsule main body 93 and the operation inputting apparatus 108 which is operated by an operator such as a surgeon for inputting an instruction signal corresponding to the operation.

The operation inputting apparatus 108 includes a direction inputting apparatus 108a that generates an instruction signal in the magnetic field direction, for example, a velocity inputting apparatus 108b that generates an instruction signal of a rotational magnetic field with a rotational frequency corresponding to an operation, and a functional button 108c that generates an instruction signal corresponding to a set function such as generation of an eccentric rotational magnetic field in response to the operation.

Next, description will be made on the capsule main body 93 including image pickup means in the insertion body to be inserted in a body cavity.

As shown in FIG. 23, the capsule main body 93 includes, on outer circumferential surface of a capsule-shaped exterior case 111, a helical protrusion (or a screw portion) 112 which is a propelling force generating structure portion that generates propelling force by rotation. Accordingly, the capsule main body 93 can be advanced and retracted in accordance with its rotational direction.

The inner portion hermetically sealed with the exterior case 111 contains an objective optical system 113, an image pickup device 114 arranged at an image-forming position, and an illumination device 115 (see FIG. 22) that emits illumination light for image pickup, and in addition, a magnet 116.

The objective optical system 113 is arranged inside a transparent hemispherical-shaped distal end cover 111a of the exterior case 111, for example, such that the optical axis of the objective optical system coincides with the central axis C of the cylindrical capsule main body 93. The center part of the distal end cover 111a serves as an observation window 117. Note that, though not shown in FIG. 23, the illumination device 115 is arranged around the objective optical system 113.

Accordingly, in this case, the field of view direction of the objective optical system 113 is along the optical axis direction of the objective optical system 113, that is, the central axis C of the cylindrical capsule main body 93.

In addition, the capsule main body 93 contains an intra-capsule coil 142 which configures the resonant circuit 140 in the inner portion in the vicinity of the rear end of the exterior case 111, for example, with the intra-capsule coil 142 oriented in a predetermined direction. More specifically, the intra-capsule coil 142 is contained wound in a solenoid shape such that the direction of the coil is set in the longitudinal direction of the capsule main body 93.

Furthermore, the magnet 116, which is arranged near the center in the longitudinal direction in the capsule main body 93, has the north pole and the south pole positioned in the direction perpendicular to the central axis C. In this case, the magnet 116 is arranged such that the center coincides with the gravity center position of the capsule main body 93. When a magnetic field is applied from outside, the center of the magnetic force exerted on the magnet 116 coincides with the gravity center position of the capsule main body 93, thereby facilitating smooth magnetic propelling of the capsule main body 93.

Moreover, the magnet 116 is arranged so as to coincide with a specific arrangement direction of the image pickup device 114. That is, when the image picked up by the image pickup device 114 is displayed, the upper direction of the image is set in the direction from the south pole toward the north pole of the magnet 116.

The magnetic field generating section 104 applies a rotational magnetic field to the capsule main body 93, thereby magnetically rotating the magnet 116. In this case, the capsule main body 93 having the magnet 116 fixed inside thereof is rotated together with the magnet 116.

At that time, the helical protrusion 112 provided on the outer circumferential surface of the capsule main body 93 contacts the inner wall of the body cavity and rotates, thereby capable of propelling the capsule main body 93. Note that the capsule main body 93 can also be retracted by rotating the capsule main body 93 in the opposite direction of the rotational direction which is the advancing direction.

When the capsule main body 93 which incorporates the magnet 116 is thus magnetically controlled by the rotational magnetic field which is an external magnetic field, it is possible to know in which direction the upper direction of the image picked up by the capsule main body 93 is oriented, from the direction of the external magnetic field.

In addition to the above-described objective optical system 113, the image pickup device 114 and the magnet 116, the capsule main body 93 includes inside thereof a signal processing circuit 120 that performs signal processing on the signal of the image picked up by the image pickup device 114, as shown in FIG. 22.

The capsule main body 93 contains inside thereof: a memory 121 that temporarily stores a digital video signal generated by the signal processing circuit 120; a wireless circuit 122 that modulates the video signal read out from the memory 121 with a high-frequency signal to convert the modulated video signal into a signal to be wirelessly transmitted, and demodulates the control signal transmitted from the image acquiring/controlling apparatus 95; a capsule controlling circuit 123 that controls the capsule main body 93 including the signal processing circuit 120 and the like; and a battery 124 for supplying an operating power supply to electric systems such as the signal processing circuit in the capsule main body 93.

Furthermore, a capacitor 141 which is electrically connected to the intra-capsule coil 142 is provided in the capsule main body 93. The capacitor 141, together with the intra-capsule coil 142, configures the resonant circuit 140.

The resonant circuit 140 is configured so as to, upon generation of an alternative magnetic field by the position/direction detecting apparatus 98, generate induced electromotive force by the alternative current magnetic field, and thereby cause a current flow through the resonant circuit 140.

Note that the coil 142 has an inherent self-resonant frequency. Accordingly, when the alternating current magnetic field having a frequency close to the self-resonant frequency is generated by the position/direction detecting apparatus 98, the coil 142 can generate effective induced electromotive force even without the capacitor 141. As a result, there is no need to provide the capacitor 141. According to such a configuration, the capacitor 141 can be omitted, thereby capable of reducing the size of the capsule main body and simplifying the configuration thereof.

In addition, as shown in FIG 22, the image acquiring/controlling apparatus 95 which wirelessly communicates with the capsule main body 93 includes a wireless circuit section 125 that wirelessly communicates with the wireless circuit 122 in the capsule main body 93 via the antenna 100.

The wireless circuit section 125 includes an image acquiring circuit 125a that acquires the signal of the image (image data) picked up by the capsule main body 93.

In addition, the image acquiring/controlling apparatus 95 incorporates inside thereof: the main processing section 102 connected to the wireless circuit section 125, which performs a display processing for displaying the image, in addition to the above-described position/direction calculating processing on the image data transmitted from the capsule main body 93; and a controlling section 127 connected to the main processing section 102, which performs various kinds of control and has a function of the inductive magnetic field deciding circuit 103.

Furthermore, the image acquiring/controlling apparatus 95 includes a storage section 128 which is connected to the controlling section 127 and which stores the information on the rotational magnetic field generated by the magnetic field generating section 104 and the information on the setting by the direction inputting apparatus 108a and the like, via the magnetic field controlling circuit 106.

Moreover, the storage section 128 includes a storing area for the specific position information storage section 128a which stores the above-described specific position information. Though the main processing section 102 is configured to be connected with the specific position information storage section 128a through the controlling section 127 in FIG. 22, the main processing section 102 may be configured to be directly connected to the specific position information storage section 128a, as shown in FIG. 20.

In addition, though FIG. 22 shows a configuration in which the inductive magnetic field deciding circuit 103 is provided in the controlling section 127, the main processing section 102 and the inductive magnetic field deciding circuit 103 may be directly connected to each other as shown in FIG. 20.

The main processing section 102 is connected with the display apparatus 107 on which the image and the like picked up by the image pickup device 114, passed through the wireless circuits 122, 125, and processed by the main processing section 102, are displayed. Furthermore, since the image is picked up with the capsule main body 93 rotated, the main processing section 102 performs a processing of correcting the orientation of the image to a certain direction at the time that the image is displayed on the display apparatus 107, thereby performing the image processing so as to display an easy-to-view image for the surgeon (disclosed in the Japanese Patent Application Laid-Open Publication No. 2003-299612).

The controlling section 127 receives instruction signals corresponding to the operations from the direction inputting apparatus 108a, the velocity inputting apparatus 108b and the like which configure the operation inputting apparatus 108, and the controlling section 127 performs controlling operation corresponding to the instruction signals.

In addition, the controlling section 127 is connected to the storage section 128 and constantly stores therein, via the magnetic field controlling circuit 106, the information on the orientation of the magnetic field (the normal line direction on the magnetic field rotational plane of the rotational magnetic field) generated in the magnetic field generating section 104 in response to the alternating current signal from the signal generating circuit 105 and the information on the orientation of the magnetic field.

After that, even when the operations to change the orientation of the rotational magnetic field and the orientation of the magnetic field are performed, the orientation of the rotational magnetic field and the orientation of the magnetic field can be continuously changed, thereby enabling a smooth change. Note that the storage section 128 may be provided in the controlling section 127.

The signal generating circuit 105, which is connected to the controlling section 127 via the magnetic field controlling circuit 106, includes three alternating current signal generating circuits 131 that generate alternating current signals and control the frequencies and the phases of the signals, and a driver section 132 composed of three drivers that amplify the alternating current signals. The output signals of the three drivers are supplied to the three electromagnets 133a, 133b and 133c which configure the magnetic field generating section 104, respectively.

In the present embodiment, the electromagnets 133a, 133b and 133c are arranged so as to generate magnetic fields in three axes directions which are perpendicular to one another. For example, each of the electromagnets 133a, 133b and 133c is a pair of opposing coils including two coils, and as these electromagnets, three axis opposing coils whose magnetic field generating directions are perpendicular to one another can be applied. Examples of the opposing coils include two Helmholtz coils arranged so as to sandwich the patient 92.

Note that the magnetic field generating section 104 may be formed with Helmholtz coils for rotational magnetic field generation as the coils for generating rotational magnetic fields to induce the capsule main body 93.

The capsule medical system 91 generates an instruction signal in the magnetic field direction by the operation of the direction inputting apparatus 108a configuring the operation inputting apparatus 108. In addition, by the operation of the velocity inputting apparatus 108b, the capsule medical system 91 generates an instruction signal of the rotational magnetic field with a rotational frequency corresponding to the operation.

Furthermore, the capsule medical system 91 generates an (alternating or cyclic) vibration magnetic field set by the operation of the functional button 108c. The rotational magnetic field thus generated can cause the magnet 116 in the capsule main body 93 to generate a couple for rotating the central axis C itself around a center point of the central axis C in the longitudinal direction of the capsule main body 93.

In this case, before the central axis C itself is completely rotated, the alternating or cyclic vibration magnetic field is applied so as to change the orientation of the vibration magnetic field (work as the couple) in the opposite direction. As a result, the capsule main body 93 is tilted or vibrated.

Note that the operator tilts a joystick not shown in a direction in which the operator desires to advance the capsule main body, and thereby the direction inputting apparatus 108a generates the rotational magnetic field so as to move the capsule main body 93 in the desired direction.

FIG. 24 shows the situation at the time that the rotational magnetic field is applied, for example. Application of the rotational magnetic field to the capsule main body 93 enables the magnet 116 incorporated in the capsule main body 93 to rotate, and the rotation enables the capsule main body 93 to advance or retract.

As shown in FIG. 24, the rotational magnetic field is applied such that the poles of the rotational magnetic field changes on the rotational magnetic field plane perpendicular to the direction of the central axis C (y' in FIG. 24) in the longitudinal direction of the capsule main body 93. This allows the capsule main body 93 to rotate around the longitudinal axis thereof together with the magnet 116 fixed in the capsule main body 93 in the direction perpendicular to the longitudinal direction.

According to the rotational direction, by engaging the capsule main body 93 with the inner wall of the body cavity using the helical protrusion 112 shown in FIG 23, the capsule main body 93 can be advanced and retracted.

FIG. 25 shows a situation at the time that the vibration magnetic field (magnetic field for couple generation) is applied to the rotational magnetic field, for example. The vibration magnetic field (magnetic field for couple generation), which works on the capsule main body 93 so as to swing (vibrate) the magnet 116 around the central axis C direction (yz in FIG. 25) in the longitudinal direction.

Accordingly, the capsule main body 93 is rotated around the central axis C in the longitudinal direction and the central axis C of the rotation is eccentrically tilted. That is, the configuration enables such a movement that a rotary torque of a rotating spinning top becomes smaller and an arbor swings due to working of the gravity force (hereinafter, such a movement is referred to as a jiggling movement).

When the capsule main body 93 is advanced or retracted in the lumen having approximately the same diameter as that of the capsule main body 93 along the longitudinal direction of the lumen, the capsule main body 93 can be smoothly moved by applying rotational magnetic field for rotating the capsule main body 93 around the longitudinal direction.

However, in the curved part of the lumen, the capsule main body 93 sometimes abuts the curved part, so that if the capsule main body 93 is rotated only around the longitudinal direction, it is sometimes difficult to smoothly move the capsule main body in the curved direction.

In such a case, as described above, vibration magnetic field is applied along the central axis C in the longitudinal direction of the capsule main body 93 such that a force works around the center of the capsule main body 93 to rotate the central axis C, thereby allowing the jiggling movement of the capsule main body 93, and when the longitudinal direction at the time of the jiggling movement coincides the curved direction of the lumen, the capsule main body 93 can be smoothly moved in the curved direction.

Note that the states of the capsule main body 93 or the rotational magnetic field are constantly grasped such that the orientation of the rotational magnetic field can be controlled to direct in a desired arbitrary direction from the current advancing direction by tilting the joystick. In the present embodiment, the state of the rotational magnetic field (specifically, the orientation of the rotational magnetic field and the orientation of the magnetic field) is constantly stored in the storage section 128.

Specifically, the instruction signal of the operation in the operation inputting apparatus 108 in FIG. 22 is inputted to the controlling section 127. The (inductive magnetic field deciding circuit 103) of the controlling section 127 outputs a control signal for generating a rotational magnetic field corresponding to the instruction signal to the magnetic field controlling circuit 106 and stores the information on the orientation of the rotational magnetic field and the orientation of the magnetic field in the storage section 128.

Accordingly, information on the rotational magnetic field generated by the magnetic field generating section 104 and the cyclically changing orientation of the magnetic field which forms the rotational magnetic field is constantly stored in the storage section 128. Note that the information to be stored in the storage section 128 is not limited to the information corresponding to the control signal from the controlling section 127 for controlling the orientation of the rotational magnetic field and the orientation of the magnetic field. Based on the control signal outputted from the controlling section 127 to the magnetic field controlling circuit 106, the alternating current signals generated in the signal generating circuit 105 and the information for deciding the orientation of the rotational magnetic field actually outputted from the magnetic field generating section 104 via the driver section 132 and the orientation of the magnetic field may be transmitted from the magnetic field controlling circuit 106 to the controlling section 127 and stored in the storage section 128.

In addition, in the present embodiment, when the application of the rotational magnetic field is started and stopped, and the orientation of the rotational magnetic field (in other words, orientation of the advancing direction of the capsule main body 93) is changed, the rotational magnetic field is controlled and continuously changed such that a force is exerted not suddenly but smoothly on the capsule main body 93.

In addition, due to the rotation of the capsule main body 93, the image picked up by the image pickup device 114 is also rotated in the present embodiment. If the image is displayed as-is on the display apparatus 107, the displayed image is also rotated, which reduces the operability of instruction operation in a desired direction by the direction inputting apparatus 108a. Therefore, it is desired to cease the rotation of the display image.

In the present embodiment, as described in the Japanese Patent Application Laid-Open Publication No. 2003-299612, the main processing section 102 or the controlling section 127 performs processing of correcting the rotated image into an image whose rotation is ceased.

Note that the image is rotated based on the information on the orientation of the magnetic field, and then the image may be displayed by canceling the rotation of the capsule main body 93 (alternatively, correlation processing and the like is performed on the image and a still image in a predetermined direction may be displayed).

As described with reference to FIG. 20, in the present embodiment, the intra-image specific position detecting section 102b detects the position of the luminal dark part in the image based on the image picked up by the image pickup means in the capsule main body 93. The generation of the magnetic field for magnetically inducing the capsule main body is controlled depending on the position of the luminal dark part or the existence or nonexistence of the luminal dark part. Even when the luminal dark part is not detected, appropriate processing is performed.

In the present embodiment, in order to deal with the case where the luminal dark part is not detected, under the management of the specific position information managing section 102c, the specific position information detected by the intra-image specific position detecting section 102b and the information on the position and the direction of the capsule main body 93 as calculation information calculated by the position/direction calculating section 102a are stored in the specific position information storage section 128a in order of time, as shown in FIG. 26, for example.

In the specific example in FIG. 26, at each of the time ti (i=1, 2, etc., m), for example, the position (ti) of the luminal dark part (as specific position information) detected from the image picked up at each of the time ti, and the position and direction (ti) of the capsule main body 93 detected at each of the time ti as calculated information by the position/direction calculating section 102a are associated with each other and stored in order of time.

When determining that the luminal dark part is not detected, the specific position information managing section 102c reads out the information stored in the specific position information storage section 128a and uses the information for inducing the capsule main body.

Note that, as described below, when the luminal dark part is no longer detected by a predetermined processing, the specific position information managing section 102c may determine the state of the image to perform a processing of deciding the inductive magnetic field.

That is, in the normal image, the luminal dark part is shown as a circular shape and the center position of the circular shape can be detected as the running direction of the lumen. On the other hand, when the lumen is flattened, the luminal dark part is shown as a line shape or a band-shaped dark part (also referred to as a dark line) in the acquired image.

In such a case, under the management of the specific position information managing section 102c, the intra-image specific position detecting section 102b detects the center position of the expansion of the dark line as the position of the luminal dark part. On the other hand, when the center of the expansion of the dark line cannot detected, the intra-image specific position detecting section 102b refers to the past information and detects the position of the luminal dark part by estimation. When the intra-image specific position detecting section 102b cannot estimate the position of the luminal dark part, the capsule main body 93 is brought back into a past state.

FIG. 27 shows examples of images in the lumen which are acquired by the capsule main body 93. The images acquired by the capsule main body 93 differ depending on the position of the capsule main body in the lumen such as the large intestine and the luminal state. The images A, B, C, D and E in FIG 27 differ from one another according to the position of the capsule main body 93 in the lumen or the luminal state and the like shown in FIG 28. Note that the states corresponding to the images A, B, C, D and E in FIG. 27 are shown with the same reference numerals A, B, C, D and E in FIG. 28.

The images A, B and C in FIG 27 are normal images suitable for detecting the dark part. On the other hand, the images D and E are the images (specific images) different from the normal images.

The image A in FIG. 27 shows the state where liquid or air is in the lumen and the distal direction of the lumen can be detected as a dark part.

The image B shows the state where liquid or air is in the lumen and the distal direction of the lumen can barely be identified as a dark part in the screen.

The image C shows the state where liquid or air is in the lumen and a space exists between the capsule and the intestinal wall, but the capsule main body 93 faces the luminal wall direction and the dark part corresponding to the running direction of the lumen cannot detected.

The image D shows the state where the distal end of the lumen is flattened, and the part where the intestinal tissue contacts can be identified but cannot be identified as a clear dark part.

The image E shows the state where the dome of the capsule main body 93 closely contacts the lumen, and the blood vessels flowing on the surface of the lumen can be identified, but only the information on the running of the lumen can be acquired.

Since the capsule main body 93 is positioned substantially at the center of the lumen in the images A and B, the information on the dark part (direction of the lumen) can be acquired. In this case, by applying propulsion force to the capsule main body 93 toward a dark part direction, the capsule main body 93 can be advanced along the lumen.

On the other hand, in the image D, the lumen is flattened and a clear dark part cannot be detected. However, in such a case, the hollow of the flattened lumen forms a slightly dark part (dark line), the brightness level of the tissues is the same on the left and right of the line (this is a point different from the image C to be described later).

The specific position information managing section 102c determines that the image in the above-described state shows the luminal state in the specific image, for example.

In addition, the specific position information managing section 102c estimates the certainty that the dark line indicates a region of the hollow of the flattened lumen by image processing, thereby determining whether the capsule main body 93 can be advanced to the center of the dark line. For example, when the width of the dark line can be calculated, the specific position information managing section 102c detects the center of the line as the position of the dark part and determines for advancing the capsule main body.

When determining to advance the capsule main body, the specific position information managing section 102c causes the inductive magnetic field deciding circuit 103 to decide an inductive magnetic field, and causes the magnetic field generating section 104 to generate a magnetic field for applying propelling force to the capsule main body 93 to advance it, through the magnetic field controlling circuit 106 and the like.

When determining not to advance the capsule main body, the specific position information managing section 102c causes a magnetic field to be generated to induce the capsule main body 93 to go back in the lumen, according to pieces of the past information (calculated by the position/direction detecting apparatus 98 and the position/direction calculating section 102a) which are stored in the specific position information storage section 128a, and which correspond to the past trajectory drawn by the capsule main body 93.

When thus determining not to advance the capsule main body, the specific position information managing section 102c causes a magnetic field to be generated to induce the capsule main body 93 to retract in the lumen according to the past trajectory (pieces of the past information calculated by the position/direction detecting apparatus 98 and the position/direction calculating section 102a) drawn by the capsule main body 93.

The specific position information managing section 102c performs control to advance the capsule main body 93 again after the dark part identifiable state (the state of image A or image B) is reached.

In addition, when the capsule main body 93 is retracted, the position where the capsule main body 93 existed forms vacancy, which sometimes brings about a state where the dark part can be identified on the image.

When the vacancy is recognized as the dark part, the same operations will be repeated. When the capsule main body 93 is retracted and detection of dark part is resumed, it is preferable to detect the dark part after the capsule main body 93 is retracted to some extent (a distance longer than the entire length of the capsule main body 93, for example).

On the other hand, in the image C, the dark part is not detected but the folds of the lumen can be identified. The deep parts of the folds of the lumen are recognized as the dark lines.

However, unlike the above-described state of the image D, the difference in the brightness of the tissues is observed on the left and right of the dark lines. Therefore, the difference from the state of the image D can be recognized.

In this case, the running direction of the lumen is estimated with reference to the past position/direction data of the capsule main body 93 and the past data of the dark part detection. The magnetic field generated by the inductive magnetic field generating apparatus 94 is controlled to make the orientation of the capsule main body 93 direct toward the estimated running direction of the lumen.

When the capsule main body 93 is directed in the running direction of the lumen by the direction change, the image becomes the state of the image A through the state of the image B, which clarifies the advancing direction.

When the dark part observable state is not reached, the direction of the capsule main body 93 is returned first based on the past specific position information of the capsule main body 93, and thereafter control may be performed to retract the capsule main body 93 according to the past trajectory of the capsule main body 93. Then the induction of the capsule main body may be started again after the dark part observable state is reached. Other operations are the same as those in the case of the image D.

In the case of the image E, the capsule main body is too close to the lumen, so that the information on the dark part (direction of the lumen) cannot be acquired, which disables the control. Accordingly, when the state of the image E is reached, it is necessary to ensure the information on the dark part (direction of the lumen).

In the image E, a clear blood vessel image is visualized. This blood vessel image can be easily detected by image processing. In this case, based on the past position/direction information of the capsule main body 93 and the past dark part information, direction changing control is performed to direct the capsule main body 93 in the running direction of the lumen. When a vacancy exists around the capsule main body 93, the orientation of the capsule main body 93 can be changed by the direction changing control, and the dark part detectable states as shown in the images A, B are reached.

However, when the capsule main body 93 is strongly restrained by the lumen even if the direction change operation is performed, the state where the direction of the capsule main body 93 cannot be changed is maintained. In this case, control to retract the capsule main body 93 is performed with reference to the past position/direction information of the capsule main body 93 and the past dark part information. The following operations are the same as in the case of the image C.

Furthermore, there may be a case where the capsule main body 93 cannot be retracted. In the case, the induction of the capsule main body 93 is stopped to bring the capsule main body 93 into an unrestrained state. This stabilizes the capsule main body 93 along and closest to the lumen. In this case, the state is as shown in the image D. Therefore, according to the control in the example of the image D, the induction can be resumed.

Next, representative operation examples according to the present embodiment will be described with reference to FIG. 29.

Description will be made on control contents in the case where the capsule main body 93 is used to pick up the images of a body cavity, particularly from an oral cavity into a lumen such as an esophagus, a small intestine, large intestine and the like.

FIG. 29 shows the control content according to the present embodiment. As shown in step S51 in FIG. 29, the capsule main body 93 picks up an image at a fixed cycle, for example, while moving in the lumen, and transmits the picked up images.

As shown in step S52, the image acquiring circuit 125a in the image acquiring/controlling apparatus 95 acquires the transmitted image. The image is inputted to the intra-image specific position detecting section 102b in the main processing section 102.

Furthermore, as shown in step S53, the position/direction detecting apparatus 98 acquires the detection signal corresponding to the position and direction of the capsule main body 93 in response to the signal from the resonant circuit 140 in the capsule main body 93.

As shown in step S54, the position/direction calculating section 102a in the main processing section 102 calculates the position and direction of the capsule main body 93 based on the detection signal.

As shown in the next step S55, the intra-image specific position detecting section 102b performs an operation to detect the position information of the luminal dark part from the image acquired by the image acquiring circuit 125a.

Furthermore, as shown in step S56, the position information of the luminal dark part and the information on the position and the direction of the capsule main body 93 are stored in the specific position information storage section 128a in order of time through the specific position information managing section 102c.

Furthermore, as shown in step S57, the specific position information managing section 102c determines the existence or nonexistence of the luminal dark part. This determination is performed by the specific position information managing section 102c by monitoring the detecting operation of the luminal dark part performed by the intra-image specific position detecting section 102b, for example.

When it has been determined that the luminal dark part exists, as shown in step S58, the inductive magnetic field deciding circuit 103 controls the magnetic field controlling circuit 106 so as to decide an inductive magnetic field generated by the magnetic field generating section 104 based on the current position information of the luminal dark part detected by the intra-image specific position detecting section 102b and information on the current position and direction of the capsule main body 93 calculated by the position/direction calculating section 102a.

In the next step S59, according to the information on the decision of the inductive magnetic field, the magnetic field generating section 104 generates a rotational magnetic field as the inductive magnetic field and controls the movement of the capsule main body 93 including the orientation thereof. Then the procedure returns to the processing in step S51.

On the other hand, in step S57, when the specific position information managing section 102c has determined that the luminal dark part does not exist, the procedure moves on to step S60. In the step S60, the specific position information managing section 102c reads out the past position information of the luminal dark part and information on the position and direction of the capsule main body 93 which are stored in the specific position information storage section 128a.

As shown in step S61, the specific position information managing section 102c refers to the read-out past specific position information, and outputs to the inductive magnetic field deciding circuit 103 the information for causing the inductive magnetic field deciding circuit to decide the inductive magnetic field for reversing the orientation of the rotational magnetic field so as to bring the capsule main body 93 back into the past position and direction at the time that the luminal dark part was detected. Then the procedure moves on to step S59 where the capsule main body 93 is magnetically induced by such an inductive magnetic field. Note that as described with reference to FIG. 27 or FIG. 28, the induction may be performed in different manners depending on the state of the acquired image in the processing in step S61.

By repeating the above-described control processings, continuous magnetic induction of the capsule main body 93 is performed, thereby causing the capsule main body to advance automatically in the body cavity.

According to the present embodiment thus operated, the capsule main body 93 can be magnetically controlled using the external magnetic field such that the capsule main body 93 is advanced smoothly in the body cavity, more specifically, along the running direction of the lumen. By smoothly propelling the capsule main body 93 along the running direction of the lumen, images can be acquired in a short time. Therefore, the surgeon can smoothly perform diagnosis and the like with reference to the acquired images.

Furthermore, in the present embodiment, description has been made on a rotational magnetic induction in which a propelling force generating section (specifically, the helical protrusion) is provided to the capsule endoscope to apply rotational magnetic field. However, no limitation is placed on the method of inducing the capsule endoscope, and the capsule endoscope may be induced by a propelling force acquired by magnetic attraction. Furthermore, the position/direction detecting apparatus is not limited to a type in which the magnetic field generated from the capsule is detected outside the body, but may be a type in which the magnetic field generated outside the body is detected by the capsule to decide the position and the direction of the capsule.

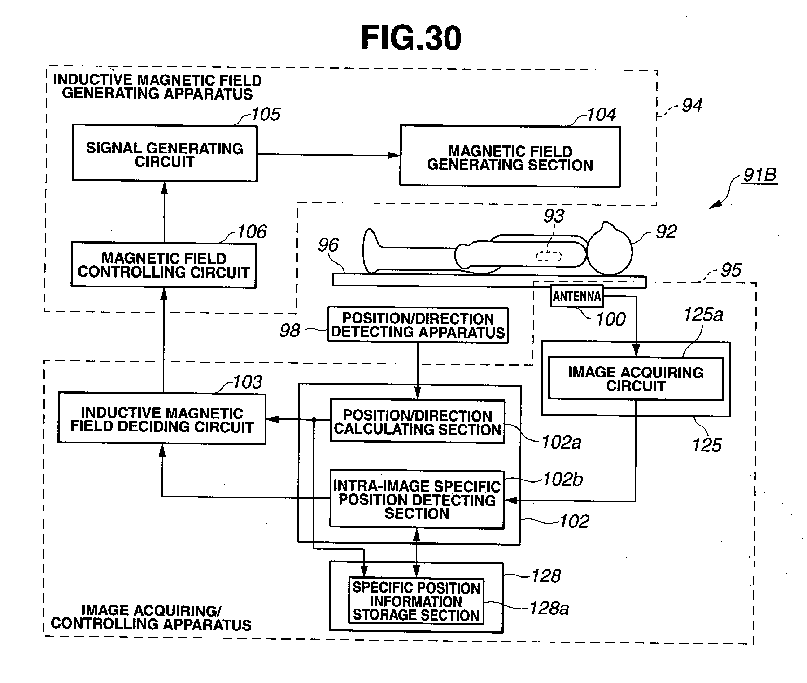

Next, a modified example of the present embodiment will be described. FIG. 30 shows a configuration of a main part of a capsule medical system 91B according to the modified example.

The capsule medical system 91 B has a configuration in which the specific position information managing section 102c is eliminated from the capsule medical system 91 in FIG. 20. When the luminal dark part is not detected, the inductive magnetic field deciding circuit 103 refers to the past information stored in the specific position information storage section 128a and decides the inductive magnetic field so as to bring the capsule back into the past state.

Alternatively, when the luminal dark part is not detected, the intra-image specific position detecting section 102b may transmit the past information stored in the specific position information storage section 128a to the inductive magnetic field deciding circuit 103 and perform a processing to bring the capsule main body back into the past state.

In FIG. 20, the position and direction information obtained by the position/direction calculating section 102a and the position information of the luminal dark part as specific position information obtained by the intra-image specific position detecting section 102b are stored in the specific position information storage section 128a through the specific position information managing section 102c. On the other hand, in the present modified example, the position and direction information obtained by the position/direction calculating section 102a and the specific position information obtained by the intra-image specific position detecting section 102b are stored in the specific position information storage section 128a, not through the specific position information managing section 102c.

In the present modified example, when the luminal dark part is detected, the control operation is the same as that in the above-described second embodiment.

That is, if operation in the present modified example is described, when the luminal dark part is detected, the operation is as shown in the steps S51 to S59 in FIG. 29.

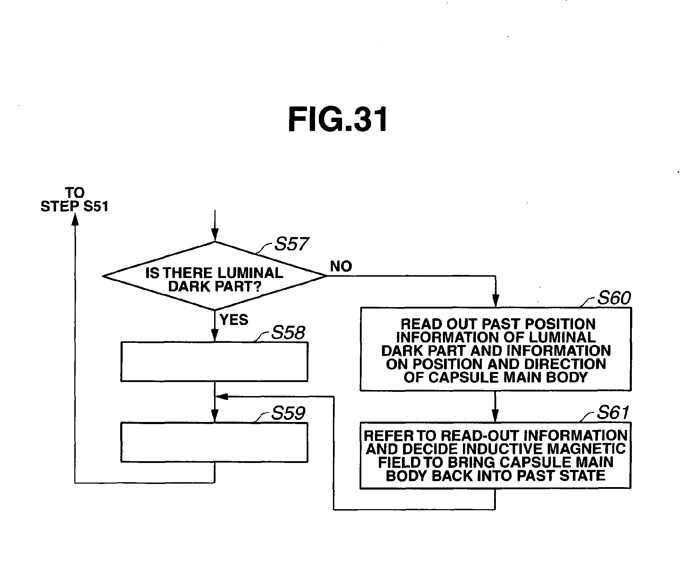

On the other hand, when the luminal dark is not detected in step S57, as in step S60 in FIG. 31, the intra-image specific position detecting section 102b reads out the past position information of the luminal dark part and information on the position and direction of the capsule main body 93 which are stored in the specific position information storage section 128a, for example.

In the next step S61', the past information stored in the specific position information storage section 128a is transmitted to the inductive magnetic field deciding circuit 103. The inductive magnetic field deciding circuit 103 refers to the transmitted information and decides inductive magnetic field so as to bring the capsule main body back into the past state. After that, the procedure moves on to step S59.

Note that, in the control processing routine in the case where the luminal dark part is not detected in step S57, the moving distance of the capsule main body 93 within a predetermined time period during the processing is calculated, and when the calculated moving distance is equal to or smaller than a threshold, generation of the inductive magnetic field may be stopped to bring the capsule main body 93 into an unrestrained state. Then, the capsule main body 93 may be moved by peristalsis of an intestinal tract and the like.

In the present modified example, the detected information of the luminal dark part is used, which can reduce the length of time for acquiring images for examination or diagnosis in the body cavity using the capsule main body 93. In addition, when the luminal dark part is not detected and it takes long to move the capsule main body, generation of the inductive magnetic field is stopped and examination in the body cavity can be performed with the capsule main body 93 using peristalsis.

Furthermore, the present modified example can simplify the image processing when performing control of the inductive magnetic field to move the capsule main body 93.

Note that, in the second embodiment and the modified example thereof, description has been made on the configuration in which the magnetic field to be applied to the capsule main body 93 is automatically controlled. However, the direction may be detected so as to insert or move the capsule main body 93 in the running direction of the body cavity and the detected direction may be displayed on the display apparatus 107 and the like.

In this case, the operator can check the direction on the display apparatus 107. In addition, when the control mode of the magnetic field is changed from the automatic control mode to the manual control mode, the movement of the capsule main body 93 may be manually prompted by operating the direction inputting apparatus 108a and the like according to the information on the direction displayed on the display apparatus 107.

Note that embodiments and the like configured by partially combining the above-described embodiments and the like also belong to the present invention.

### Industrial Applicability

A system of the present invention incorporates an image pickup apparatus in an insertion portion or an insertion body configured to be inserted in a body cavity, detects position information of the dark part corresponding to the running direction of the body cavity from the image picked up by image pickup means to calculate the insertion direction, and records the position information and the like in a time-sequential manner. Under the condition in which the detection accuracy of the position information is lowered, the past position information is allowed to be referred to. Accordingly, it is possible to smoothly insert the insertion portion and the like toward the deep part in the body cavity, thereby enabling smooth examination and diagnosis.

## Claims

1. An endoscope system comprising:
an endoscope for picking up an image in a body cavity by image pickup means provided in a distal end of an insertion portion;
position detecting means for detecting, based on luminal information acquired by the image pickup means, position information used for inserting the distal end of the insertion portion;
recording means for recording, in a time-sequential manner, the position information detected by the position detecting means;
determining means for determining whether or not the detecting operation of the position information performed by the position detecting means satisfies a set condition; and
direction calculating means for, when the determination result shows that the set condition is not satisfied, reading out the position information recorded in the recording means and outputting information on a direction in which the distal end of the insertion portion is to be inserted.

2. The endoscope system according to claim 1, wherein the determining means is dark part determining means for determining, as the condition, whether a dark part corresponding to a running direction of the body cavity exists in the luminal information.

3. The endoscope system according to claim 1 or 2, further comprising amount-of-twist detecting means for detecting an amount of twist of the insertion portion around a longitudinal axis, wherein the recording means records, in a time-sequential manner, the amount-of-twist in association with the position information.

4. The endoscope system according to any one of claims 1 to 3, further comprising position/direction detecting means for detecting a position and a direction of the distal end of the insertion portion, wherein the recording means records the information on the position and the direction in association with the position information.

5. The endoscope system according to any one of claims 1 to 3, further comprising an insertion portion distal end direction changing section for changing the direction of the distal end of the insertion portion, wherein the insertion portion distal end direction changing section changes a direction of a position of the insertion portion.

6. The endoscope system according to claim 5, wherein, when the endoscope is a capsule endoscope, the insertion portion distal end direction changing section magnetically changes the direction of the distal end of the insertion portion.

7. The endoscope system according to claim 5, further comprising a display apparatus for displaying the direction of the distal end of the insertion portion.

8. The endoscope system according to any one of claims 1 to 4, wherein the direction calculating means is bending information calculating means for calculating bending information including at least a bending direction, the information being used for bending a bending portion provided near the distal end of the insertion portion such that the distal end of the insertion portion is directed in the direction of the position based on the position information detected by the position detecting means.

9. The endoscope system according to claim 8, further comprising:
electric bending driving means for electrically bending the bending portion; and
driving control means for performing driving control to electrically drive the electric bending driving means based on an output from the bending information calculating means.

10. The endoscope system according to claim 8, further comprising display means for displaying the bending information including at least the bending direction which is calculated by the bending information calculating means.

11. The endoscope system according to claim 2, wherein the dark part determining means determines existence or nonexistence of the dark part based on information on a color tone or an edge included in the luminal information.

12. The endoscope system according to claim 1, wherein the endoscope is a capsule endoscope.

13. The endoscope system according to claim 12, further comprising:
magnetic field induction controlling means for magnetically inducing and controlling the capsule endoscope; and
managing means for managing information as to whether or not to generate an inductive magnetic field by the magnetic field induction controlling means using the position information recorded in the recording means, depending on the determination result by the determining means.

14. An image pickup system comprising:
an image pickup section provided in an insertion body configured to be inserted in a body cavity, for picking up an image in the body cavity;
luminal information detecting means for detecting luminal information corresponding to a running direction of the body cavity, from the image picked up by the image pickup section;
recording means for recording, in a time-sequential manner, luminal information detected by the luminal information detecting means;
estimating means for estimating a position and a direction of the image pickup section;
determining means for determining whether or not the detecting operation of the luminal information performed by the luminal information detecting means satisfies a set condition;
direction calculating means for, when the determining means determines that the condition is not satisfied, reading out the luminal information recorded in the recording means and calculating information on a direction in which the insertion body is moved based on the luminal information and an estimation result acquired by the estimating means; and
controlling means for controlling the direction in which the insertion body is moved, based on the information calculated by the direction calculating means.

15. The image pickup system according to claim 14, wherein the image pickup section is a capsule endoscope contained in the insertion body formed in a capsule shape.

16. The image pickup system according to claim 15, wherein the capsule endoscope contains a magnet, and the controlling means controls the direction in which the insertion body is moved by controlling an external magnetic field generated by a magnetic field generating apparatus that applies the external magnetic field for magnetically inducing the capsule containing the magnet.

17. A capsule medical system comprising:
a capsule medical apparatus including inside an image pickup section and a magnet;
an inductive magnetic field generating apparatus arranged outside of the capsule medical apparatus, for inducing the capsule medical apparatus;
a position/direction detecting apparatus for detecting a position and a direction of the capsule medical apparatus;
estimating means for estimating a moving direction based on an image acquired by the capsule medical apparatus;
recording means for recording, in a time-sequential manner, information on the position and the direction detected by the position/direction detecting apparatus and information on the moving direction estimated by the estimating means; and
inductive magnetic field controlling means for controlling an inductive magnetic field generated by the inductive magnetic field generating apparatus to move the capsule medical apparatus in the body cavity based on the estimation result by the estimating means and the detection result by the position/direction detecting apparatus.

18. The capsule medical system according to claim 17, further comprising managing means for determining whether or not the estimating means can estimate the moving direction under a set condition, and for managing, depending on the determination result, information as to whether or not to generate the inductive magnetic field by the inductive magnetic field controlling means, using the information recorded in the recording means.

19. An image processing apparatus comprising:
an inputting section for inputting an endoscopic image picked up by image pickup means provided in a distal end portion of an insertion portion configured to be inserted in a body cavity;
position detecting means for performing a processing of detecting, from the endoscopic image, position information used for introducing the distal end of the insertion portion;
recording means for recording, in a time-sequential manner, the position information detected by the position detecting means;
determining means for performing determining processing as to whether or not the processing of detecting the position information performed by the position detecting means satisfies a set condition; and
direction calculating means for, when the determining means determines that the condition is not satisfied, reading out position information recorded in the recording means and outputting information on a direction in which the distal end of the insertion portion is to be inserted.

20. The image processing apparatus according to claim 19, wherein the determining means is dark part determining means for performing a processing to determine, as the condition, existence of a dark part corresponding to the running direction of the body cavity in the endoscopic image.
